(19) Europäisches Patentamt
European Patent Office
Office européen des brevets

(11) Veröffentlichungsnummer: **0 003 532**
**B1**

(12) # EUROPÄISCHE PATENTSCHRIFT

(45) Veröffentlichungstag der Patentschrift: **28.04.82**

(51) Int. Cl.³: **C 07 C 103/84, A 61 K 31/195**

(21) Anmeldenummer: **79100217.3**

(22) Anmeldetag: **25.01.79**

(54) **Omega-(2-(N-Niederalkyl-benzamido)-phenyl)-alkansäuren, ihre Verwendung und Herstellung sowie sie enthaltende Arzneimittel.**

(30) Priorität: **03.02.78 LU 79008**

(43) Veröffentlichungstag der Anmeldung:
**22.08.79 Patentblatt 79/17**

(45) Bekanntmachung des Hinweises auf die Patenterteilung:
**28.04.82 Patentblatt 82/17**

(84) Benannte Vertragsstaaten:
**BE CH DE FR GB IT LU NL SE**

(56) Entgegenhaltungen:
**FR - A - 2 307 528**
**FR - M 5735**

(73) Patentinhaber: **Byk Gulden Lomberg Chemische Fabrik GmbH**
**Byk-Gulden-Strasse 2**
**D-7750 Konstanz (DE)**

(72) Erfinder: **Klemm, Kurt, Dr.**
**Im Weinberg 2**
**D-7753 Allensbach (DE)**
Erfinder: **Krüger, Uwe, Dr.**
**NeuhauserstraBe 11**
**D-7750 Konstanz (DE)**
Erfinder: **Rapp, Erich, Dr.**
**Irisweg 5**
**D-7760 Radolfzell 15 (DE)**
Erfinder: **Wolf, Horst, Dr.**
**BrandesstraBe 29**
**D-7750 Konstanz (DE)**
Erfinder: **Kraas, Ekkehard, Dr.**
**Lindenweg 11**
**D-7753 Allensbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 532 B1

Omega-(2-(N-Niederalkyl-benzamido)-phenyl)-alkansäuren, ihre Verwendung und Herstellung sowie sie enthaltende Arzneimittel

Die Erfindung betrifft ω-[2-(N-Niederalkyl-benzamido)-phenyl]-alkansäuren, ihre Verwendung und Herstellung sowie sie enthaltende Arzneimittel.

Aus der FR—A— 2 307 528 sind blutzuckersenkende p-substituierte Phenylalkansäuren der Formel

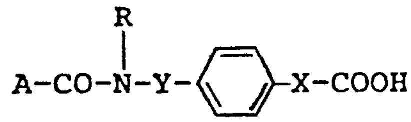

bekannt, deren wirksamste Vertreter monosubstituierte Amide (R = H) mit einer Ethylenbrücke (Y = $—CH_2—CH_2—$) zwischen dem Stickstoffatom und dem Phenylrest sind.

o-Benzamidophenylalkansäuren mit analgetischer und antiinflammatorischer Wirkung werden in der britischen Patentschrift GB—A— 1 082 466 beschrieben. Gemäß D. J. Drain et al. [J. Pharm.Pharmac. 22 (1970) 684—693] besitzen o-Benzamidophenylpropionsäuren bemerkenswerte entzündungshemmende Eigenschaften. Überraschenderweise wurde nun gefunden, daß N-alkylierte o-Benzamidophenylalkansäuren wertvolle pharmakologische Wirkungen aufweisen, die sich von denen der nicht N-alkylierten Verbindungen deutlich unterscheiden.

Gegenstand der Erfindung sind ω-[2-(N-Niederalkyl-benzamido)-phenyl]-alkansäuren der allgemeinen Formel I

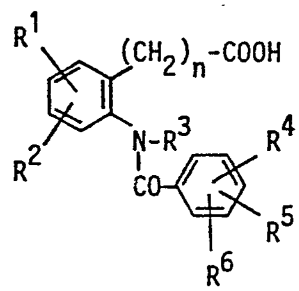

(I),

worin

n eine positive ganze Zahl von 2 bis 5 bedeutet,

$R^1$ ein Wasserstoffatom, ein Halogenatom, eine Niederalkylgruppe, eine Niederalkoxygruppe, eine Niederalkylmercaptogruppe, eine Trifluormethylgruppe oder eine gegebenenfalls durch ein Halogenatom oder eine Niederalkoxygruppe substituierte Phenylgruppe bedeutet,

$R^2$ ein Wasserstoffatom, eine Halogenatom, eine Niederalkylgruppe oder eine Niederalkoxygruppe bedeutet,

$R^3$ eine Niederalkylgruppe bedeutet,

$R^4$ ein Wasserstoffatom, ein Halogenatom, eine Hydroxygruppe, eine Niederalkylgruppe, eine Niederalkoxygruppe, eine Niederalkylmercaptogruppe, eine gegebenenfalls durch ein Halogenatom oder eine Nitrogruppe substituierte Phenylgruppe, eine Nitrogruppe, eine gegebenenfalls niederalkylierte Aminogruppe, eine Niederalkylcarbonylgruppe, eine gegebenenfalls durch ein Halogenatom substituierte Benzoylgruppe, eine Trifluormethylgruppe, eine Trifluormethoxygruppe oder eine Trifluormethylmercaptogruppe bedeutet,

$R^5$ ein Wasserstoffatom, ein Halogenatom, eine Niederalkylgruppe oder eine Niederalkoxygruppe bedeutet,

$R^6$ ein Wasserstoffatom, eine Niederalkylgruppe oder eine Niederalkoxygruppe bedeutet,

und ihre Salze mit anorganischen oder organischen Basen.

Als Halogenatome kommen Fluor, vorzugsweise Brom oder Chlor, insbesondere Chlor in Betracht. Niederalkyl-, Niederalkoxy- bzw. Niederalkylmercaptogruppe sind solche, die 1 bis 5 Kohlenstoffatome enthalten. Als Niederalkylgruppen seien genannt geradkettige Alkylreste, wie der Methyl-, Ethyl-, Propyl-, Butyl- oder Pentylrest, von denen die mit 1 bis 2 Kohlenstoffatomen bevorzugt sind, und verzweigte Alkylreste, wie der Isopropyl-, Isobutyl-, sek.-Butyl- oder Neopentylrest, von denen die mit 4 Kohlenstoffatomen bevorzugt sind. Bevorzugte Niederalkoxy- bzw. Niederalkylmercaptogruppen sind die Methoxy- bzw. Methylmercaptogruppe.

Als Salze kommen solche mit anorganischen oder organischen Basen in Betracht. Pharmakologisch nicht verwendbare oder nicht verträgliche Salze werden nach an sich bekannten Methoden in pharmakologisch, d.h. biologisch verwendbare oder verträgliche Salze übergeführt, die unter den erfindungsgemäßen Salzen bevorzugt sind. Als Kationen für die Salzbildung werden vor allem die Kationen der Alkalimetalle, Erdalkalimetalle oder Erdmetalle ver wendet, es kommen jedoch auch die entsprechenden Kationen organischer Stickstoffbasen, wie Amine, Aminoalkanole, Aminozucker, basische Aminosäuren etc. zur Anwendung.

Beispielsweise seien die Salze von Lithium, Natrium, Kalium, Magnesium, Calcium, Aluminium, Ethylendiamin, Dimethylamin, Diethylamin, Morpholin, Piperidin, Piperazin, N-Niederalkyl-(z.B. Methyl)-piperazinen, Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin Lysin, Ornithin, Arginin genannt.

Bei den Ausgesaltungen der Erfindung werden die Formeln, Substituenten und Symbole zur besseren Unterscheidung durch einen oder mehrere Sternchen gekennzeichnet.

Eine Ausgestaltung der erfindungsgemäßen Verbindungen sind $\omega$-[2-(N-Niederalkyl-benzamido)-phenyl]-alkansäuren der allgemeinen Formel 1*

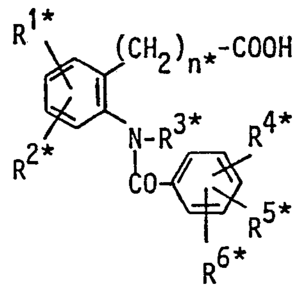

(I*),

worin

n* eine positive ganze Zahl von 2 bis 5 bedeutet,

$R^{1*}$ ein Wasserstoffatom, ein Chloratom, ein Bromatom, eine Niederalkylgruppe, eine Methoxygruppe oder eine Phenylgruppe bedeutet,

$R^{2*}$ ein Wasserstoffatom, ein Chloratom, ein Bromatom, eine Methylgruppe oder eine Methoxygruppe bedeutet,

$R^{3*}$ eine geradkettige Niederalkylgruppe bedeutet,

$R^{4*}$ ein Wasserstoffatom, ein Fluratom, ein Chloratom, ein Bromatom, eine Niederalkylgruppe, eine Hydroxygruppe, eine Niederalkoxygruppe, eine Phenylgruppe, eine Nitrogruppe, eine Aminogruppe, eine Dimethylaminogruppe oder eine Triflourmethylgruppe bedeutet,

$R^{5*}$ ein Wasserstoffatom, ein Chloratom, eine Niederalkylgruppe oder eine Niederalkoxygruppe bedeutet,

$R^{6*}$ ein Wasserstoffatom, eine Niederalkylgruppe oder eine Niederalkoxygruppe bedeutet,

und ihre Salze mit anorganischen oder organischen Basen.

Eine weitere Ausgestaltung der erfindungsgemäßen Verbindungen sind solche der allgemeinen Formel I**

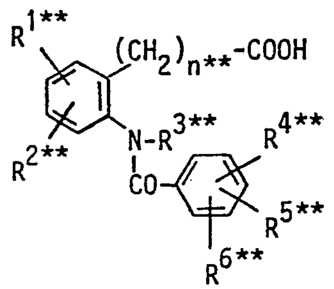

(I**),

worin

n** eine positive ganze Zahl von 2 bis 5 bedeutet,

$R^{1**}$ ein Wasserstoffatom, ein Methylgruppe, eine Methoxygruppe, ein Chloratom, ein Bromatom oder eine Phenylgruppe bedeutet,

$R^{2**}$ ein Wasserstoffatom, ein Chloratom oder eine Methylgruppe bedeutet,

$R^{3**}$ eine geradkettige Alkylgruppe mit 1 bis 4 Kohlenstoffatomen bedeutet,

$R^{4**}$ ein Wasserstoffatom, ein Chloratom, ein Bromatom, eine Methylgruppe, eine Niederalkoxygruppe, eine Phenylgruppe oder eine Trifluormethylgruppe bedeutet,

$R^{5**}$ ein Wasserstoffatom, ein Chloratom, eine Niederalkylgruppe oder eine Methoxygruppe bedeutet,

$R^{6**}$ ein Wasserstoffatom, eine Niederalkylgruppe oder eine Methoxygruppe bedeutet,

und ihre Salze mit anorganischen oder organischen Basen.

Bevorzugte erfindungsgemäß Verbindungen sind solche der allgemeinen Formel I***

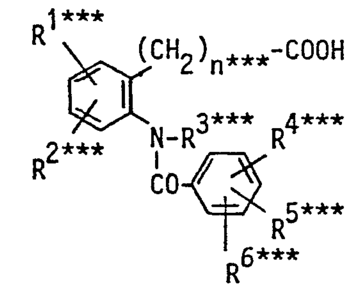

(I***),

worin

$n^{***}$ 2 oder 3 bedeutet,
$R^{1***}$ ein Wasserstoffatom, ein Chloratom, eine Methylgruppe oder eine Methoxygruppe bedeutet,
$R^{2**}$ ein Wasserstoffatom oder eine Methylgruppe bedeutet,
$R^{3***}$ eine Methylgruppe oder eine Ethylgruppe bedeutet,
$R^{4***}$ ein Wasserstoffatom, ein Chloratom, eine Phenylgruppe oder eine Trifluormethylgruppe bedeutet,
$R^{5***}$ ein Wasserstoffatom oder ein Chloratom bedeutet,
$R^{6***}$ ein Wasserstoffatom bedeutet,

und ihre pharmakologisch verträglichen Salze mit anorganischen oder organ schen Basen.

Besonders bevorzugte erfindungsgemäße Verbindungen sind solche der allgemeinen Formel I****

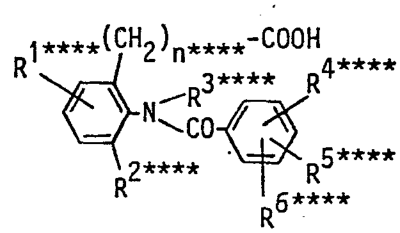

(I****),

worin

$n^{****}$ 2 oder 3 bedeutet,
$R^{1****}$ ein Wasserstoffatom, ein Chloratom, eine Methylgruppe oder eine Methoxygruppe bedeutet,
$R^{2****}$ ein Wasserstoffatom bedeutet,
$R^{3****}$ eine Methylgruppe bedeutet,
$R^{4****}$ ein Wasserstoffatom, ein Chloratom oder eine Trifluormethylgruppe bedeutet,
$R^{5****}$ ein Wasserstoffatom oder ein Chloratom bedeutet,
$R^{6****}$ ein Wasserstoffatom bedeutet,

und ihre pharmakologisch verträglichen Salze mit anorganischen oder organischen Basen.

Als Beispiele erfindungsgemäßer Verbindungen seien die folgenden Säuren und Salze genannt:

3-[5-Brom-2-(N-ethyl-2,4-dichlorbenzamido)-phenyl]-propionsäure,
3-[2-(N-n-Propyl-4-methoxybenzamido)-6-trifluormethylphenyl]-propionsäure,
3-[5-n-Butyl-2-(N-n-butyl-4-nitrobenzamido)-phenyl]-propionsäure,
3-[6-Ethyl-2-(N-ethyl-2-phenylbenzamido)-phenyl]-propionsäure,
3-[6-Methylmercapto-2-(N-methyl-3,4,5-trimethoxybenzamido)-phenyl]-propionsäure,
4-[4-Methyl-2-(N-n-butyl-4-methylmercapto-benzamido)-phenyl]-buttersäure,
4-[2-(N-n-Propyl-3-trifluormethylbenzamido)-phenyl]-buttersäure,
4-[2-(N-n-Butyl-4-phenylbenzamido)-phenyl]-buttersäure,
4-[2-(N-Methyl-4-acetylbenzamido)-5-methoxyphenyl]-buttersäure,
4-[2-(N-n-Pentyl-2-methyl-3-nitrobenzamido)-phenyl]-buttersäure,
4-[2-(N-Ethyl-2,4,5-trimethylbenzamido)-phenyl]-buttersäure,
Triethanolammonium-4-[2-(N-methyl-2,4-dichlorbenzamido)-phenyl]-butyrat.

Als erfindungsgemäße Verbindungen, die sich durch interessante Wirkungen auszeichnen, seien genannt

3-[2-(N-Methyl-4-chlor-benzamido)-phenyl]-propionsäure,
3-[2-(N-Methyl-3,4-dichlor-benzamido)-phenyl]-propionsäure,
4-[2-(N-Methyl-4-chlorbenzamido)-6-methoxyphenyl]-buttersäure,
4-[2-(N-Methyl-3,4-dichlorbenzamido)-5-methoxyphenyl]-buttersäure,
5-[2-(N-Methyl-3,4-dichlorbenzamido)-phenyl]-valeriansäure,

insbesondere

3-[2-(N-Methyl-benzamido)-phenyl]-propionsäure,
4-[2-(N-Methyl-4-chlor-benzamido)-phenyl]-buttersäure,
4-[2-(N-Methyl-2,4-dichlorbenzamido)-phenyl]-buttersäure,

3-[2-(N-Methyl-3,4-dichlorbenzamido)-5-chlorphenyl]-propionsäure,
3-[2-(N-Methyl-3-trifluormethylbenzamido)-5-chlorphenyl]-propionsäure,
4-[2-(N-Methyl-3,4-dichlorbenzamido)-phenyl]-buttersäure,
4-[2-(N-Methyl-3-trifluormethylbenzamido)-phenyl]-buttersäure,
4-[2-(N-Methyl-benzamido)-phenyl]-buttersäure,
und ihre pharmakologisch verträglichen Salze.

Die erfindungsgemäßen Verbindungen weisen wertvolle pharmakologische Eigenschaften auf, die sie gewerblich verwertbar machen. Sie wirken hypoglycämisch und hemmen die Glukosebildung in der Leber.

Aufgrund ihrer vorteilhaften Wirksamkeit sind die ω-[2-(N-Niederalkylbenzamido-phenyl]-alkansäuren I, ihre Salze bzw. ihre Ausgestaltungen I*, I**, I*** und I**** zur Behandlung und Prophylaxe von Krankheiten, die auf Störungen des Glucosestoffwechsels beruhen, geeignet. Beispielsweise werden behandelt prädiabetische Zustände zur Verhinderung der Manifestation des Diabetes, manifester Diabetes, z.B. Erwachsenendiabetes, labiler Diabetes von Jugendlichen. Ferner werden die erfindungsgemäßen Verbindungen eingesetz zur Prophylaxe von koronaren, cerebralen und peripheren Durchblutungsstörungen, diabetischer Angio- oder Retinopathie.

Die erfindungsgemäßen Verbindungen werden daher zur Behandlung und Prophylaxe von Krankheiten, die auf Störungen des Glucosestoffwechsels beruhen, eingesetzt. Gegenstand der Erfindung sind somit auch die erfindungsgemäßen Verbindungen zur Amwendung bei der Bekämpfung der oben angegebenen Krankheiten. Ebenso umfaßt die Erfindung die Verwendung der erfindungsgemäßen Verbindungen zur Herstellung von Arzneimitteln, die zur Bekämpfung der angeführten Krankheiten eingesetzt werden.

Ein weiterer Gegenstand der Erfindung sind Arzneimittel, die eine oder mehrere der ω-[2-(N-Niederalkyl-benzamido)-phenyl]-alkansäuren der allgemeinen Formel I

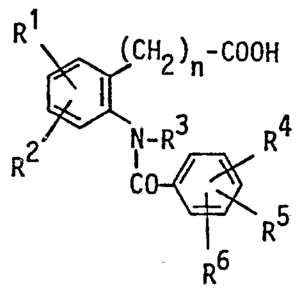

(I),

worin

n eine positive ganze Zahl von 2 bis 5 bedeutet,

$R^1$ ein Wasserstoffatom, ein Halogenatom, eine Niederalkylgruppe, eine Niederalkoxygruppe, eine Niederalkylmercaptogruppe, eine Trifluormethylgruppe oder eine gegebenenfalls durch ein Halogenatom oder eine Niederalkoxygruppe substituierte Phenylgruppe bedeutet,

$R^2$ ein Wasserstoffatom, ein Halogenatom, eine Niederalkylgruppe oder eine Niederalkoxygruppe bedeutet,

$R^3$ eine Niederalkylgruppe bedeutet,

$R^4$ ein Wasserstoffatom, ein Halogenatom, eine Hydroxygruppe, eine Niederalkylgruppe, eine Niederalkoxygruppe, eine Niederalkylmercaptogruppe, eine gegebenenfalls durch ein Halogenatom oder eine Nitrogruppe substituierte Phenylgruppe, eine Nitrogruppe, eine gegebenenfalls niederalkylierte Aminogruppe, eine Niederalkylcarbonylgruppe, eine gegebenenfalls durch ein Halogenatom substituierte Benzoylgruppe, eine Trifluormethylgruppe, eine Trifluormethoxygruppe oder eine Trifluormethylmercaptogruppe bedeutet,

$R^5$ ein Wasserstoffatom, ein Halogenatom, eine Niederalkylgruppe oder eine Niederalkoxygruppe bedeutet,

$R^6$ ein Wasserstoffatom, eine Niederalkylgruppe oder eine Niederalkoxygruppe bedeutet,

und/oder ihre pharmakologisch verträglichen Salze mit anorganischen oder organischen Basen enthalten.

Ausgestaltungen der Arzneimittel sind solche, die ω-[2-(N-Niederalkylbenzamido)-phenyl]-alkansäuren der Formel I*, I**, I***, I**** oder ihrer bevorzugten Vertreter und/oder ihre pharmakologisch verträglichen Salze mit anorganischen oder organischen Basen enthalten.

Die Arzneimittel werden nach an sich bekannten Verfahren hergestellt. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 1 bis 95, vorzugsweise 15 bis 85 Gewichtsprozent der Gesamtmischung.

Die erfindungsgemäßen Verbindungen können im humanmedizinischen Bereich in beliebiger Form, z.B. systemisch, angewandt werden unter der Voraussetzung, daß die Ausbildung bzw. Aufrechterhaltung von ausreichenden Blut- oder Gewebsspiegeln an Wirkstoffen gewährleistet ist. Das

kann beispielsweise durch orale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion oder einer Suspension sein.

Unter "Einheitsdosis" wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachem einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die Arzneimittel gemäß der Erfindung enthalten, wenn sie in Einheitsdosen vorliegen und für die Applikation, z.B. am Menschen bestimmt sind, etwa 10 bis 1000 mg, vorteilhafterweise 50 bis 500 mg und insbesondere 100 bis 300 mg Wirkstoff.

Im allgemeinen hat es sich in der Humanmedizin als vorteilhaft erwiesen, den oder die Wirkstoffe bei oraler Gabe in einer Tagesdosis von etwa 0,3 bis etwa 150 vorzugsweise 1,5 bis 75, insbesondere 3 bis 15 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von etwa 0,1 bis etwa 50, vorzugsweise 0,5 bis 25, insbesondere 1 bis 5 mg/kg Körpergewicht.

Bei einer parenteralen Behandlung, z.B. einer intravenösen oder intramusculären Applikation können ähnliche Dosierungen zur Anwendung kommen. Bei dieser Therapie werden etwa 1 bis etwa 5 mg Wirkstoff/kg Körpergewicht appliziert.

Die therapeutische Verabreichung der pharmazeutischen Zubereitung erfolgt bei Dauermedikation im allgemeinen zu festgelegten Zeitpunkten, wie 1 bis 4 mal am Tage, z.B. jeweils nach den Mahlzeiten und/oder am Abend. Bei akuten Anlässen erfolgt die Medikation zu variiendem Zeitpunkt. Unter bestimmten Gegebenheiten kann es erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittles sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe nimmt der Fachmann aufgrund seines Fachwissens vor.

Die pharmazeutischen Zubereitungen bestehen in der Regel aus den erfindungsgemäßen Wirkstoffen und nichttoxischen, pharmazeutische verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wäßrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Xylit; Granulierungs- und Verteilungsmittel, z.B. Calciumphosphat oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß eine verzögerte Auflösung und Resorption des Arzneimittels im Gastrointestinaltrakt und damit z.B. eine bessere Verträglichkeit, Protrahierung oder eine Retardierung erreicht wird. Gelatinekapseln können den Arzneistoff vermischt mit einem festen Verdünnungsmittel, z.B. Calciumcarbonat oder Kaolin, oder einem öligen Verdünnungsmittel, z.B. Paraffinöl, enthalten.

Wäßrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzunmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharin, Natriumcyclamat, enthalten.

Ölige Suspensionen können z.B. Paraffinöl und Verdickungsmittel, wie Bienenwachs, Hartparaffin oder Cetylalkohol, enthalten; ferner Süßungsmittel, Geschmacksmittel und Antioxidantien.

Im Wasser dispergierbare Pulver und Granulate können die Arzneistoffe in Mischung mit Dispergier-, Benetzungs- und Suspendiermitteln, z.B. den obengenannten, sowie mit Süßungsmitteln, Geschmacksmitteln und Farbstoffen enthalten.

Emulsionen können z.B. Paraffinöl neben Emulgiermitteln, wie Akaziengummi, Traganthgummi, Phosphatiden, Sorbitanmonooleat, Polyoxyethylensorbitanmonooleat, und Süßungs- und Geschmacksmitteln enthalten.

Zur parenteralen Anwendung der Arzneistoffe dienen steril injizierbare wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglycol, enthalten können.

Der oder die Wirkstoffe können gegebenenfalls mit einem oder mehreren der angegebenen Träger- oder Zusatzstoffe auch in mikroverkapselter Form formuliert werden.

Neben den erfindungsgemäßen ω-[2-(N-Niederalkyl-benzamido)-phenyl]-alkansäuren, in denen die Substituenten die oben angegebene Bedeutung haben, und/oder ihren Salzen können die pharmazeutischen Zubereitungen weiterhin einen oder mehrere pharmakologisch aktive Bestandteile anderer Arzneimittelgruppen, wie Antidiabetika (Sulfonamide, Sulfonylharnstoffe u.a.), z.B. Carbutamid, Tolbutamid, Chlorpropamid, Glibenclamid, Glibornurid, Glisoxepid, Gliquidon, Glymidin oder Hypolipidämika, wie Nikotinsäure sowie deren Derivate und Salze enthalten.

Gegenstand der Erfindung ist auch ein Verfahren zur Herstellung der ω-[2-(N-Niederalkylbenzamido)-phenyl]-alkansäuren der allgemeinen Formel I bzw. der Ausgestaltung I*, I**, I***, I**** und ihrer Salze mit anorganischen oder organischen Basen. Das Verfahren ist dadurch gekennzeichnet, daß man

a) eine ω-(2-Niederalkylamino-phenyl)-alkansäure der allgemeinen Formel II

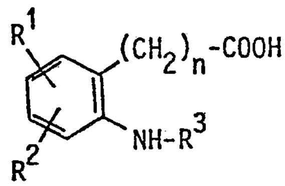

(II),

worin

n, $R^1$, $R^2$ und $R^3$ die oben angegebene Bedeutung haben, oder ein Salz davon mit einem Benzoylderivat der allgemeinen Formel II

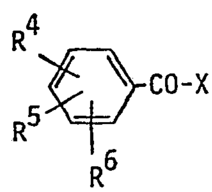

(III),

worin

X ein Halogenatom bedeutet und $R^4$, $R^5$ und $R^6$ die oben angegebene Bedeutung haben, in basischer Lösung acyliert und gewünschtenfalls anschließend erhaltene freie Säuren in ihre Salze oder erhaltene Salze in die freien Säuren überführt oder

b) eine ω-[2-(N-Niederalkyl-benzamido)-phenyl]-alkylverbindung der allgemeinen Formel IV

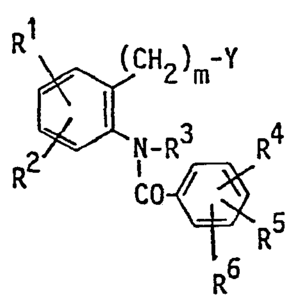

(IV),

worin

$R^1$, $R^2$, $R^3$, $R^4$, $R^5$ und $R^6$ die oben angegebene Bedeutung haben, m eine positive ganze Zahl von 1 bis 4 und Y eine Fluchtgruppe bedeuten, mit einem Dialkylmalonat, vorzugsweise einem Diniederalkylmalonat, in Gegenwart basischer Kondensationsmittel umsetzt und den erhaltenen substituierten Malonester verseift und decarboxyliert und gewünschtenfalls anschließend erhaltene freie Säuren in ihre Salze oder erhaltene Salze in die freien Säuren überführt oder

c) zur Herstellung von Verbindungen der Formel I, worin n = 2 ist, eine 2-(N-Niederalkyl-benzamido)-zimtsäure der allgemeinen Formel V

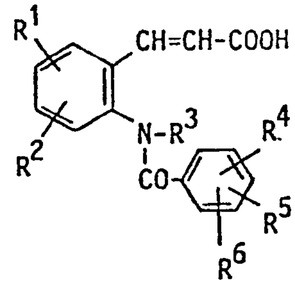

(V),

worin

$R^1$, $R^2$, $R^3$, $R^4$, $R^5$ und $R^6$ die oben angegebene Bedeutung haben, oder ein Salz oder einen Niederalkylester davon hydriert und gegebenenfalls anschließend erhaltene $\omega$-[2-(N-Niederalkyl-benzamido)-phenyl]-propionsäureniederalkylester in die freie Säuren oder deren Salze überführt.

Die Acylierung gemäß Verfahrensvariante a) wird nach an sich bekannten Methoden durchgeführt. Sie erfolgt beispielsweise durch Vorlage der wäßrigen basischen Lösung der frisch hergestellten Ausgangsverbindungen II und Zugabe der Benzoylderivate III. Dies schließt jedoch nicht aus, daß gegebenenfalls noch andere Lösungsmittel oder Lösungsvermittler, wie Ether, z.B. Diethylether oder Tetrahydrofuran oder Diether von Polyolen, z.B. Dioxan oder Diglyme, oder Ketone, z.B. Aceton, oder Amide, z.B. Dimethylformamid, oder gegebenenfalls halogenierte Kohlenwasserstoffe, z.B. Methylenchlorid, Benzol, Toluol, Cyclohexan, Petrolether, oder weitere aprotische Lösungsmittel, wie Dimethylsulfoxid oder Acetonitril, zugesetzt werden. Ferner können die Säuren II, vorzugsweise in Form eines Salzes, auch in nicht wässerigen Lösungsmitteln, z.B. Ethern, Ketonen, Amiden, oder gegebenenfalls halogenierten Kohlenwasserstoffen, suspendiert und in Gegenwart von Basen mit den Benzoylderivaten III, vorzugsweise den Benzoylchloriden, zur Reaktion gebracht werden.

Basische Lösungen werden vorwiegend unter Verwendung von Alkalien, z.B. Lithium-, Natrium- oder Kaliumhydroxid, Natriumhydrogencarbonat, Natrium- oder Kaliumcarbonat hergestellt. Es können dazu jedoch auch die entsprechenden Erdalkalimetallverbindungen, z.B. Calcium- oder Bariumhydroxid, oder organische Basen, wie Tetramethylammoniumhydroxid, eingesetzt werden. Die eingesetzte Base muß geeignet sein, den pH-Wert der Lösung auf 7,2 bis 10, vorzugsweise 8 bis 9, einzustellen, um unerwünschte Nebenreaktionen zu vermeiden, sonst ist die Art der eingesetzten Base von geringerer Bedeutung. Obwohl die basischen Lösungen im allgemeinen umgehend der Acylierung zugeführt werden, können sie bei Bedarf, z.B. zur Bereitung von Vorratslösungen, längere Zeit vor der Acylierung aufbereitet werden.

Die Reaktionszeiten bzw. -temperaturen liegen im Bereich von 0,5 bis 10 Stunden bzw. 0 bis 40°C; wird die Reaktion bei Raumtemperatur durchgeführt, ist sie im allgemeinen innerhalb 1 bis 2 Stunden abgeschlossen.

Die Umsetzung gemäß Verfahrensweise b) erfolgt in an sich bekannter Weise. Als Fluchtgruppe (=Abgangsgruppe) Y kommen die bei nucleophilen Substitutionen üblichen Fluchtgruppen, wie Halogenatome, z.B. ein Chlor- oder Bromatom, oder Alkylsulfonyloxy- oder Benzolsulfonyloxygruppen, z.B. eine Mesyloxy- oder eine p-Tolylsulfonyloxygruppe, in Betracht. Die Reaktion der Verbindungen IV mit den Dialkylmalonaten erfolgt nach üblichen Malonestersynthesen; so werden beispielsweise die Verbindungen IV in wasserfreien Lösungsmitteln, wie Niederalkanolen (vorzugsweise Ethanol), Amiden, gegebenenfalls halogenierten Kohlenwasserstoffen, Ketonen, Ethern, Dimethylsulfoxid oder Acetonitril, mit dem Anion eines Dialkylmalonats umgesetzt. Das Anion des Dialkylmalonats wird vorzugsweise in situ hergestellt, z.B. aus Natriumethylat und Diethylmalonat. Es kann jedoch auch in Form eines vorher hergestellten Alkalimetallsalzes, z.B. als Natriummalonester, eingesetzt werden. Der erhaltene substituierte Malonester wird in üblicher Weise verseift, z.B. mit alkoholischem Kaliumhydroxid. Die so erhaltene freie Dicarbonsäure wird nach bekannten Methoden decarboxyliert, z.B. durch Erhitzen auf Temperaturen von 150 bis 200°C. Verseifung und Decarboxylierung werden üblicherweise getrennt ausgeführt. Dies schließt jedoch ihre Durchführung als Eintopfreaktion nicht aus.

Die Hydrierung gemäß Verfahrensvariante c) wird nach an sich bekannten Methoden durchgeführt. Sie erfolgt beispielsweise durch Hydrierung eines entsprechenden Zimtsäureesters in Gegenwart eines Palladium-Kohle-Katalysators und nachfolgender Hydrolyse des Esters zur entsprechenden Propionsäure (vgl. Organic Synthesis, Coll. Vol. IV, 408). Ebenso läßt sich eine Zimtsäure V in schwach alkalischer Lösung mit Hydrazin in Gegenwart katalytischer Mengen von Raney-Nickel reduzieren [N.S. Hjelte, Chem. Scand. 15(1961)1200]. Alternativ wird bei der Hydrierung ein entsprechendes Zimtsäurealkalimetallsalz in wäßrigbasischer Lösung mit Natriumamalgam nach E. Fischer [in Fieser/Fieser, Reagents for Organic Synthesis, Vol. I, S.1031, Wiley and Sons New York (1967)] eingesetzt. Die gleichen Salze werden auch durch elektrolytische Reduktion in die erfindungsgemäßen Verbindungen I übergeführt (s. Organic Synthesis, Coll. Vol. I, 311). Bei der Hydrierung werden zweckmäßigerweise lediglich solche Ausgangsverbindungen V eingesetzt, deren Substituenten nicht durch die Hydrierung angegriffen werden; beispielsweise werden Nitrogruppen bei Hydrierung reduziert.

Die überführung der freien Säuren in die Salze bzw. der Salze in die freien Säuren erfolgt nach dem Fachmann bekannten Methoden. Salze können beispielsweise gewonnen werden, indem man freie Säuren der allgemeinen Formel I bzw. der Ausgestaltungen I*, I**, I*** bzw. I**** mit dem stöchiometrischen Äquivalent einer entsprechenden Base umsetzt oder leicht lösliche Salze durch doppelte Umsetzung in schwer lösliche Salze überführt oder beliebige Salze in pharmakologische verträgliche Salze überführt. Freie Säuren I werden durch Umsetzung der Salze mit Mineralsäuren, z.B. Salzsäure, und Aufarbeitung erhalten.

Die für das erfindungsgemäße Verfahren a) einzusetzenden Phenylalkansäuren II werden durch basische Hydrolyse entsprechender N-Niederalkyl-lactame X in wäßrigen oder nichtwäßrigen Medien gewonnen, die durch Alkylierung der entsprechenden Lactame IX mit Alkylierungsmitteln $R^3$-Z zugänglich sind. Die für die Herstellung der Phenylalkansäuren II (worin n = 2 ist ) benötigten Tetrahydrochinolin-2-one (IX, n = 2) werden durch Friedel-Crafts-Alkylierung von entsprechenden $\beta$-Chlorpropionaniliden (VI) erhalten.

Die für Phenylalkansäuren II (worin n = 3, 4 oder 5 ist) benötigten 2-Azabenzo-[c]-cycloheptanone (IX, n = 3) bzw. -cyclooctanone (IX, n = 4) bzw. -cyclononanone (IX, n = 5) werden durch Ringerweiterung entsprechender Benzo-[b]-cyclohexanone (VII, n = 3) bzw. -cycloheptanone (VII, n = 4) bzw. -cyclooctanone (VII, n = 5) hergestellt, z.B. mit Hilfe der Schmidt-Reaktion (mit Natriumazid) oder durch Beckmann-Umlagerung (der entsprechenden Ketoxime VIII). Es ergibt sich folgendes Reaktionsschema:

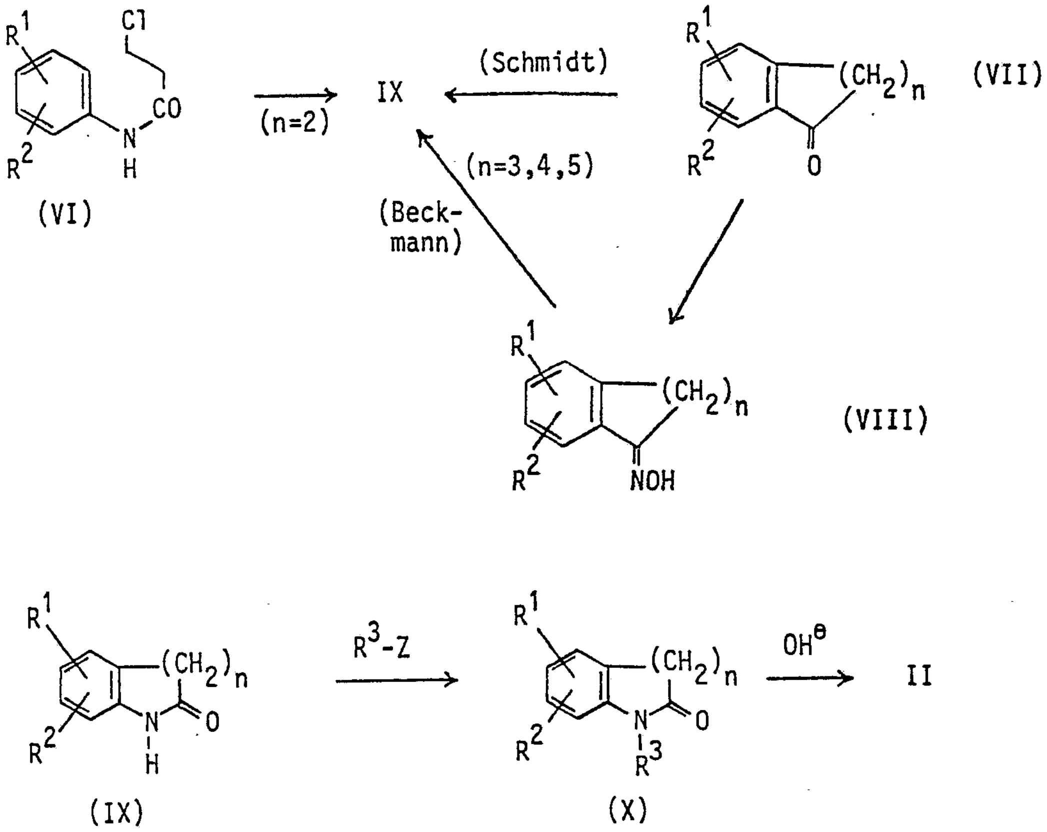

(VI) (VII) (VIII) (IX) (X)

Alternativ werden die Phenylalkansäuren II (n = 3, 4 oder 5) hergestellt durch Umsetzung von gegebenenfalls substituiertem 2-Nitrotoluol XI mit einer Lithiumbase, z.B. n-Butyllithium, anschließende Reaktion mit einem $\omega$-Halogenalkancarbonsäureniederalkylester XII, worin Hal ein Chlor- oder Bromatom und $R^7$ eine Niederalkylgruppe bedeutet, katalytische Hydrierung des Produktes XIII (Umwandlung der Nitro- in eine Aminogruppe) und anschließende Alkylierung nach bekannten Methoden. Bei der Alkylierung wird die Estergruppe verseift, so daß man eine Lösung von II erhält, die unmittelbar weiter verwendet werden kann.

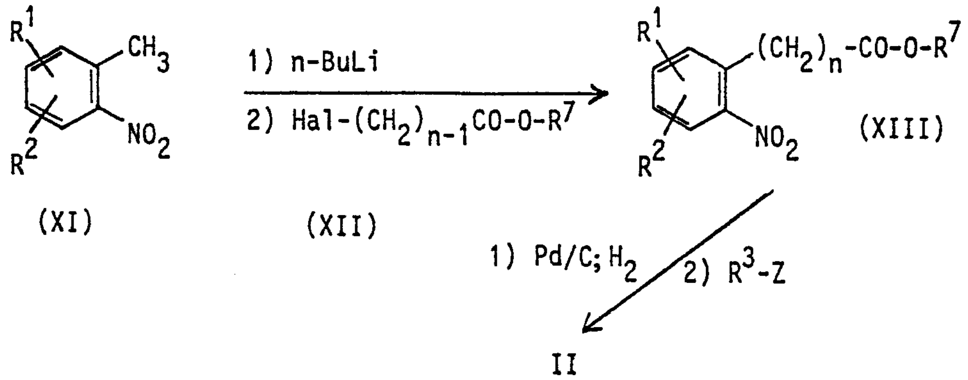


Die Friedel-Crafts-Alkylierung der $\beta$-Chlorpropionanilide VI bzw. die Ringerweiterung der Benzocycloalkanone VII zu den Lactamen IX erfolgen nach bekannten Verfahren. Zur Alkylierung werden die Lactame IX entweder unter Feuchtigkeitsausschluß in inerten Lösungsmitteln, wie Ethern, Dimethylformamid, Toluol, mit Natriumamid bzw. Natriumhydrid oder mit Natronlauge und anschließend mit den üblichen Alkylierungsmitteln $R^3$-Z, worin $R^3$ eine Niedrigalkylgruppe und Z eine Fluchtgruppe bedeuten, umgesetzt. Als Alkylierungsmittel seien u.a. genannt Methyliodid, Dimethylsulfat, Ethyliodid, Propylbromid, Isobutylbromid, Neopentyliodid. Die N-Niederalkyllactame X werden durch kräftiges Erhitzen in basischer Lösung (Temperaturen von 100° bis 160°C, gegebenenfalls unter Einsatz eines Autoklaven) in die Ausgangsverbindungen II übergeführt. Zweckmäßigerweise werden die Verbindungen II nach Entfernen überschüssigen Lösungsmittels ohne Isolierung mit den Benzoylderivaten III zu den erfindungsgemäßen Verbindungen weiter umgesetzt, da so unerwünschte Ausbeuteverluste vermieden werden. Ferner können die zur Hydrolyse der N-Niederalkyllactame X eingesetzten basischen Lösungen gleichzeitig zur Neutralisation der bei der Acylierung freiwerdenden Halogenwasserstoffe dienen.

Gegenstand der Erfindung ist daher auch ein Verfahren zur Herstellung der Verbindungen I und ihrer Salze, das dadurch gekennzeichnet ist, daß man ein N-Niederalkyllactam X in basischer Lösung hydrolysiert und mit einem Benzoylderivat III acyliert und gewünschtenfalls anschließend erhaltene freie Säuren oder Salze ineinander überführt.

Diese vorteilhafte Ausführung schließt jedoch nicht aus, daß gewünschtenfalls die $\omega$-(2-Niederalkylamino-phenyl)-alkansäuren II nach der Hydrolyse in Form der Salze isoliert werden und in einem gesonderten Schritt acyliert werden, wobei gegebenenfalls andere basische Lösungen eingesetzt werden.

Zur Herstellung der Verbindungen der Ausgestaltungen I*, I**, I*** bzw. I**** werden entsprechende Ausgangsmaterialien II*, II**, II*** bzw. II****, III*, III**, IIII*** bzw. III****, IV*, IV**, IV*** bzw. IV****, V*, V**, V*** bzw. V****, X*, X**, X*** bzw. X****, worin die Substituenten die oben angegebene Bedeutung haben, umgesetzt.

Die für das erfindungsgemäße Verfahren einsetzbaren Benzoylderivate III sind dem Fachmann bekannt oder können nach bekannten Verfahren hergestellt werden. Unter den Säurehalogeniden III sind die -chloride bevorzugt. Die Ausgangsverbindungen V sowie die Vorprodukte sind ebenfalls bekannt oder können nach bekannten Verfahren hergestellt werden. Die Ausgangsverbindungen IV für die Verfahrensvariante b) werden ebenfalls nach bekannten Verfahren erhalten. So werden sie beispielsweise aus nach den Verfahrensvarianten a) oder c) erhaltenen Alkansäuren I durch Reduktion der —COOH-Gruppe zur $CH_2$—OH-Gruppe und deren anschließende Umwandlung in die —$CH_2$—Y-Gruppe (z.B. durch Halogenierung oder Tosylierung) hergestellt.

Die folgenden Beispiele dienen zur Veranschaulichung der Erfindung. Die Angabe der Temperaturen erfolgt in °C, Fp. bedeutet Schmelzpunkt, Kp. Siedepunkt (bei . . . Torr).

## BEISPIELE

### Beispiel 1

3-[2-(N-Methylbenzamido)-phenyl]-propionsäure

$$R^3 = -CH_3,\ R^1 = R^2 = R^4 = R^5 = R^6 = -H,\ n = 2$$

a) Eine gemäß b) frisch hergestellte wäßrig-basische Lösung von 3-[2-(N-Methylamino)-phenyl]-propionsäure wird mit 2 N Salzsäure auf pH 8,5 eingestellt. Eine Lösung von 9,0 g Benzoylchlorid in 50 ml Diethylether wird unter Rühren tropfenweise dazugegeben, wobei der pH der Reaktionsmischung durch Zusatz von 1 N Natronlauge konstant auf 8,5 gehalten wird. Nach einer weiteren Stunde Rühren wird die wäßrige Phase abgetrennt und zweimal mit je 100 ml Diethylether gewaschen. Danach wird mit 2 N Salzsäure auf pH 1—2 angesäuert und mit Methylenchlorid (5mal je 200 ml) extrahiert. Die Extrakte werden über Natriumsulfat getrocknet und eingedampft. Der Rückstand wird aus Essigsäureethylester umkristallisiert und ergibt 11,9 g der Titelverbindung (66% d.Th.), Fp. 106—107°.

b) 10,4 g N-Methyl-1,2,3,4-tetrahydrochinolin-2-on werden mit 12,2 g Kaliumhydroxid in 45 ml Ethylenglykolmonoethylether 3,5 Stunden auf Rückfluß erhitzt. Nach dem Abkühlen wird mit 400 ml Wasser verdünnt und zweimal mit je 200 ml Diethylether extrahiert. Die organischen Extrakte werden verworfen. Die frisch bereitete Lösung wird ohne weitere Aufarbeitung unter a) eingesetzt.

### Beispiel 2

3-[2-(N-Methyl-3,4-dichlorbenzamido)-phenyl]-propionsäure

$$R^3 = -CH_3,\ R^4 = R^5 = -Cl,\ R^1 = R^2 = R^6 = -H,\ n = 2$$

a) Zu einer gemäß b) erhaltenen Lösung von 3-[2-(N-Methylamino)-phenyl]-propionsäure wird unter Rühren eine Lösung von 13,4 g 3,4-Dichlorbenzoylchlorid in 50 ml Diethylether zugetropft, wobei durch Zusatz von 1 N Natronlauge der pH konstant auf 8,5 gehalten wird. Weitere Aufarbeitung wie in Beispiel 1 a) führt zu 14,3 g der Titelverbindung (63% d.Th.), Fp. 149—150°C.

b) 10,4 g N-Methyl-1,2,3,4-tetrahydrochinolin-2-on werden zusammen mit 16,6 g Bariumhydroxid und 200 ml Wasser in einem Rührautoklaven 16 Stunden auf 150° erhitzt. Nach Abkühlen wird Wasser zugegeben (ca 400 ml) und mit Kohlendioxid überschüssiges Bariumsalz gefällt; dabei darf der pH nicht unter 7,5 sinken. Der Niederschlag wird abfiltriert; zum Filtrat wird solange 10%ige Natriumcarbonatlösung zugesetzt, bis pH 8,5 erreicht ist. Nach erneuter Filtration wird die Lösung ohne weitere Aufarbeitung unter a) eingesetzt.

N-Methyl-1,2,3,4-tetrahydrochinolin-2-on wird durch N-methylierung von 1,2,3,4-Tetrahydrochinolin-2-on mit Dimethylsulfat analog Beispiel 3 b) als zähes Öl erhalten.

### Beispiel 3

3-[5-Chlor-2-(N-methyl-2-phenylbenzamido)-phenyl]-propionsäure

$$R^1 = -Cl,\ R^3 = -CH_3,\ R^4 = \text{Phenyl},\ R^2 = R^5 = R^6 = -H,\ n = 2$$

a) Die gemäß b) bereitete wäßrig-basische Lösung von 3-(5-Chlor-2-methylamino-phenyl)-propionsäure wird analog Beispiel 1 a) auf pH 8,5 eingestellt und mit 6,6 g 2-Phenylbenzoylchlorid umgesetzt und aufgearbeitet. Umkristallisation aus Essigsäureethylester liefert 7,1 g der Titelverbindung (Ausbeute 56% d.Th.).

b) 17,6 g 3-Chlorpropion-4-chloranilid wird zusammen mit 32,4 g Aluminiumchlorid 3 Stunden auf 110° erhitzt. Die noch heiße Flüssigkeit wird mit 600 ml Eiswasser verrührt. Nach Versetzen mit 20 ml 2 N Salzsäure wird dreimal mit je 200 ml Essigsäureethylester extrahiert. Trocknen der organischen Phase über Natriumsulfat, Einengen und Umkristallisieren aus Essigsäureethylester/Petrolether (1:1) ergibt 10,2 g 6-Chlor-1,2,3,4-tetrahydrochinolin-2-on (Ausbeute 70% d.Th.), Fp. 165—167°.

Dieses Tetrahydrochinolinon wird in 100 ml Dimethylformamid/Toluol (1:1) gelöst; nach Zugabe von 1,8 g Natriumhydrid (80% in Paraffinöl) wird 30 Minuten bei Raumtemperatur gerührt, danach werden 7,8 g Dimethylsulfat zugegeben und 1 Stunde auf 50° erwärmt. Nach Abkühlen wird die Mischung mit 600 ml Wasser und 20 ml 2 N Natronlauge versetzt, 10 Minuten gerührt und dann mit 2 N Salzsäure angesäuert. Extraktion mit Essigsäureethylester (4mal 300 ml), Trocknen und Einengen ergibt festen Rückstand, der aus Essigsäureethylester/Petrolether (1:1) umkristallisert wird und 6,0 g (55% d.Th.) 6-Chlor-1-methyl-1,2,3,4-tetrahydrochinolin-2-on (Fp. 76—78°) ergibt.

6,0 g 6-Chlor-1-methyl-1,2,3,4-tetrahydrochinolin-2-on werden analog Beispiel 1 b) hydrolysiert. Die so bereitete Lösung wird analog 1 b) aufgearbeitet und dann unter a) weiter umgesetzt.

Beispiel 4
4-[2-(N-Methyl-4-chlorbenzamido)-phenyl]-buttersäure

$$R^3 = -CH_3,\ R^4 = -Cl,\ R^1 = R^2 = R^5 = R^6 = -H,\ n = 3$$

a). Die gemäß b) erhaltene wäßrig-basische Lösung der 4-(2-N-Methylaminophenyl)-buttersäure wird analog Beispiel 1 a) mit 6,3 g 4-Chlorbenzoylchlorid in 30 ml Diethylether acyliert und aufgearbeitet. Man erhält nach Umkristallisation aus Essigsäureethylester:Petrolether (1:1) 6,1 g der Titelverbindung (Ausbeute 51% d.Th.), Fp. 123—124°.

b) 30,0 g $\alpha$-Tetralonoxim werden in 100 ml Pyridin gelöst und unter Eiskühlung langsam mit 100 ml Phosphoroxidchlorid in 250 ml Pyridin versetzt. Es wird 2 Stunden bei 0° gerührt, dann wird die Lösung mit 300 ml Chloroform verdünnt. Das Gemisch wird vorsichtig auf 1 kg Eis und 400 ml konzentrierte Salzsäure gegossen (heftige Reaktion!). Nach Rühren bis zur Beendigung der Reaktion wird die organische Phase abgetrennt. Die wässerige Phase wird mit 3mal 300 ml Chloroform extrahiert; die vereinigten Extrakte werden über Natriumsulfat getrocknet, eingeengt und aus Ethanol umkristallisiert. Ausbeute 17,4 g (59% d.Th.) 2-Aza-benzo[c]-cycloheptanon (Fp. 141—142°).

Dieses Cycloheptanon wird analog Beispiel 3 b) mit Natriumhydrid und Dimethylsulfat N-methyliert und ergibt nach Aufarbeitung und Umkristallisation aus Essigsäureethylester/Petrolether (1:3) 17,3 g (Ausbeute 92% d.Th.) 2-Aza-2-methylbenzo[c]-cycloheptanon.

6,3 g 2-Aza-2-methyl-benzo[c]-cycloheptanon werden analog Beispiel 1 b) mit 8,1 g Kaliumhydroxid in 40 ml Ethylenglykolmonoethylether hydrolysiert, aufgearbeitet (d.h. vom Ethylenglykolmonoethylether befreit) und dann für die Reaktion gemäß a) eingesetzt.

Beispiel 5
3-[2-(N-Methyl-4-chlorbenzamido)-phenyl]-propionsäure

$$R^3 = -CH_3,\ R^4 = -Cl,\ R^1 = R^2 = R^5 = R^6 = -H,\ n = 2$$

Analog Beispiel 1 a) wird eine wäßrig-basische Lösung von 6,5 g Kalium-3-[2-(N-methylamino)-phenyl]-propionat mit 4-Chlorbenzoylchlorid acyliert. Nach Aufarbeitung und Umkristallisation aus Essigsäureethylester erhält man 5,3 g (Ausbeute 56% d.Th.) der Titelverbindung (Fp. 156—158°).

Beispiel 6
4-[2-(N-Methylbenzamido)-phenyl]-buttersäure

$$R^3 = -CH_3,\ R^1 = R^2 = R^4 = R^5 = R^6 = -H,\ n = 3$$

a) Die gemäß b) bereitete wäßrig-basische Lösung der 4-(2-Methylaminophenyl)-buttersäure wird mit 200 ml Wasser verdünnt und mit 2 N Salzsäure auf pH 8,5 eingestellt. Analog Beispiel 4 a) wird tropfenweise mit Benzoylchlorid versetzt unter Einhaltung von pH 8,5 durch Zugabe von Natronlauge. Umkristallisation aus Essigsäureethylester/Petrolether (1:2) ergibt 7,3 g (Ausbeute 48% d.Th.) der Titelverbindung (Fp. 101°).

b) 9,0 g 2-Aza-2-methyl-benzo[c]-cycloheptanon werden in 200 ml 6 N Natronlauge 60 Stunden unter Rückfluß erhitzt; nach Abkühlen wird mit 2mal 150 ml Diethylether gewaschen. Die so bereitete Lösung wird unter a) weiter umgesetzt.

Beispiel 7
4-[2-(N-Methyl-3-trifluormethylbenzamido)-phenyl]-buttersäure

$$R^3 = -CH_3,\ R^4 = -CF_3,\ R^1 = R^2 = R^5 = R^6 = -H,\ n = 3$$

Analog Beispiel 6 b) und a) werden 4,5 g 2-Aza-2-methylbenzo[c]-cycloheptane in 6 N Natronlauge hydrolysiert und anschließend bei pH 8,5 mit 3-Trifluormethylbenzoylchlorid acyliert. Nach Umkristallisation aus Petrolether erhält man 2,7 g (Ausbeute 30% d.Th.) der Titelverbindung (Fp. 84—85°).

Beispiel 8
4-[2-(N-Ethyl-2,4-dichlorbenzamido)-phenyl]-buttersäure

$$R^3 = -CH_2-CH_3,\ R^4 = R^5 = -Cl,\ R^1 = R^2 = R^6 = -H,\ n = 3$$

a) Der gemäß b) bereitete Kalium-4-[2-(N-ethylamino)-phenyl]-butyrat enthaltende Rückstand wird in 100 ml Wasser gelöst; nach Einstellen des pH's der Lösung auf 8,2 (mit 2 N Salzsäure) werden 3,5 g 2,4-Dichlorbenzoylchlorid in 50 ml Diethylether zugetropft. Durch gleichzeitige Zugabe von 1 N Natronlauge wird der pH der Lösung konstant auf 8,2 gehalten. Aufarbeitung und Umkristallisation aus

Essigsäureethylester/Petrolether (1:1) ergibt 3,1 g der Titelverbindung (Ausbeute 48% d.Th.) als Öl.

b) 3,1 g 2-Aza-2-ethyl-benzo[c]-cycloheptanon werden in 20 ml 5 N Kaliumhydroxid 60 Stunden unter Rückfluß erhitzt. Nach Abkühlen wird die Lösung zur Trockne eingedampft; der feste Rückstand, der noch überschüssiges Kaliumhydroxid enthält, wird mit Diethylether gewaschen und ohne weitere Reinigung in a) eingesetzt.

Beispiel 9

3-[4-Chlor-2-(N-methyl-4-chlorbenzamido)-phenyl]-propionsäure

$$R^3 = -CH_3,\ R^1 = R^4 = -Cl,\ R^2 = R^5 = R^6 = -H,\ n = 2$$

Analog Beispiel 1 b) und a) werden 5,2 g 7-Chlor-1-methyl-1,2,3,4-tetrahydrochinolin-2-on hydrolysiert und durch Waschen mit Diethylether von Ethylenglykolmonoethylether befreit. Die auf pH 8,6 eingestellte wässerigbasische Lösung der 3-(4-Chlor-2-methylamino-phenyl)-propionsäure wird mit 4,6 g 4-Chlorbenzoylchlorid acyliert; nach entsprechender Aufarbeitung erhält man 6,8 g der Titelverbindung als zähes Öl.

Beispiel 10

3-[5-Chlor-2-(N-methyl-3-trifluormethylbenzamido)-phenyl]-propionsäure

$$R^1 = Cl,\ R^3 = -CH_3,\ R^4 = -CF_3,\ R^2 = R^5 = R^6 = -H,\ n = 2$$

Eine nach Beispiel 3 b) bereitete Lösung von 6 g 3-(5-Chlor-2-methylaminophenyl)-propionsäure wird analog Beispiel 1 a) bei pH 8,5 mit 6,2 g 3-Trifluormethylbenzoylchlorid in 30 ml Dioxan umgesetzt und aufgearbeitet. Nach Umkristallisation aus Essigsäureethylester/Petrolether (1:1) werden 6,4 g (55% d.Th.) der Titelverbindung, Fp. 124—126° erhalten.

Beispiel 11

3-[5-Chlor-2-(N-methyl-3,4-dichlorbenzamido)-phenyl]-propionsäure

$$R^1 = R^4 = R^5 = -Cl,\ R^3 = -CH_3,\ R^2 = R^6 = -H,\ n = 2$$

a) 6,2 g 5-Chlor-2-(N-methyl-3,4-dichlorbenzamido)-zimtsäuremethylester werden in 200 ml Essigsäureethylester gelöst und nach Zugabe von ca. 0,5 g 10% Palladiumkohle-Katalysator in einer Umlaufapparatur bei Normaldruck hydriert. Nach Filtration wird das Lösungsmittel im Vakuum entfernt; der Rückstand wird mit 150 ml 5%iger Kaliumhydroxidlösung (in Methanol) behandelt. Nach 24 Stunden wird eingeengt und extraktiv aufgearbeitet. Man erhält 5,4 g der Titelverbindung, Fp. 159—160°.

b) 12,8 g 2-Acetamido-5-chlorzimtsäuremethylester werden in 200 ml Dimethylformamid/Toluol (1:1) gelöst und mit 1,6 g 80%igem Natriumhydrid in Paraffinöl versetzt; nach 30 Minuten Rühren werden bei 0° 7 g Dimethylsulfat zugetropft. Man läßt 2 Stunden bei Raumtemperatur rühren, setzt 500 ml Wasser und 50 ml 2 N Natronlauge zu und wäscht mit Toluol. Nach Ansäuern wird mit Methylenchlorid extrahiert; nach Entfernen des Lösungsmittels und chromatographischer Reinigung verbleiben 7,6 g 5-Chlor-2-(N-methyl-acetamido)-zimtsäure als Öl. Dieses Öl wird mit 100 ml 5 N Salzsäure 24 Stunden bei ca. 100° gerührt; nach Abkühlen wird mit verdünnter Natronlauge auf pH 8,5 eingestellt und mit 10 g Natriumhydrogencarbonat versetzt. Unter Rühren werden 7,5 g 3,4-Dichlorbenzoylchlorid in 50 ml Toluol zugetropft. Aufarbeitung wie unter Beispiel 1 a) ergibt 10,8 g 5-Chlor-2-(N-methyl-3,4-dichlorbenzamido)-zimtsäure, die ohne weitere Reinigung mit 200 ml Methanol unter Zusatz von 4 ml konzentrierter Schwefelsäure 2 Tage bei Raumtemperatur umgesetzt werden. Nach Extraktion mit Methylenchlorid und Aufarbeitung erhält man 8,3 g des entsprechenden Methylesters, der gemäß a) umgesetzt wird.

Beispiel 12

Benzylammonium-3-[2-(N-methyl-3,4-dichlorbenzamido)-phenyl]-propionat

$$R^3 = -CH_3,\ R^4 = R^5 = -Cl,\ R^1 = R^2 = R^6 = -H,\ n = 2$$

2,2 g 3-[2-(N-Methyl-3,4-dichlorbenzamido)-phenyl]-propionsäure werden in 15 ml Diethylether und 10 ml Aceton gelöst. Nach Zugabe von 0,8 g Benzylamin und kurzem Rühren wird die Lösung eingedampft. Der zähflüssige Rückstand wiord mehrmals mit Diethylether gewaschen, widersetzt sich jedoch Kristallisationsversuchen. Man erhält 2,85 g der Titelverbindung als erstarrtes Öl.

Beispiel 13

3-[2-(N-Ethyl-3,4-dichlorbenzamido)-5-methyl-phenyl]-propionsäure

$R^1$ = —$CH_3$, $R^3$ = —$CH_2$—$CH_3$, $R^4$ = $R^5$ = —Cl, $R^2$ = $R^6$ = —H, n = 2

a) Eine in Analogie zu Beispiel 3 b) aus 4,7 g 1-Ethyl-6-methyl-1,2,3,4-tetrahydrochinolin-2-on bereitete wäßrig-basische Lösung von 3-(2-Ethylamino-5-methyl-phenyl)-propionsäure wird gemäß Beispiel 1 a) mit 5,2 g 3,4-Dichlorbenzoylchlorid in 30 ml Methylenchlorid umgesetzt und aufgearbeitet. Nach Umkristallisation aus Diethylether/Petrolether (1:3) erhält man 2,1 g (22% d.Th.) der Titelverbindung, Fp. 107—109°.

b) Zu 9,6 g 6-Methyl-1,2,3,4-tetrahydrochinolin-2-on in 150 ml Methylenchlorid gibt man unter Rühren 1,4 g Benzyltriethylammoniumchlorid (TEBA) und eine Lösung von 12 g Ätznatron in 12 ml Wasser. Nach 20 Minuten werden 23,2 g Diethylsulfat langsam dazugetropft; man läßt 20 Stunden rühren, davon die letzten 4 Stunden unter Rückfluß. Durch Zugabe von 100 ml 4 n Natronlauge wird überschüssiges Diethylsulfat zersetzt. Darauf wird angesäuert und mehrmals mit Methylenchlorid extrahiert. Nach Trocknen und Eindampfen der organischen Phase wird der Rückstand an Kieselgel chromatographiert (Laufmittel: Methylenchlorid). Man erhält 9,4 g 1-Ethyl-6-methyl-1,2,3,4-tetrahydrochinolin-2-on als Öl (83% d.Th.).

Beispiel 14

Ammonium-3-[5-methyl-2-(N-methyl-3-trifluormethyl-benzamido)-phenyl]-propionat

$R^1$ = $R^3$ = —$CH_3$, $R^4$ = $CF_3$, $R^2$ = $R^5$ = $R^6$ = —H, n = 2

a) Eine gemäß b) hergestellte wäßrig-basische Lösung (pH 8,0) von 5,3 g 3-(2-Methylamino-5-methylphenyl)-propionsäure wird im Vakuum zur Trockne eingedampft. Nach azeotroper Entfernung von Wasserspuren durch Abdampfen mit Toluol wird der Rückstand in 150 ml Dimethylformamid suspendiert und zuerst mit 2,8 g Triethylamin, dann tropfenweise unter Rühren mit 6,0 g 3-Trifluormethylbenzoylchlorid versetzt. Nach 12 Stunden Rühren wird zwischen Wasser und Diethylether verteilt und gemäß Beispiel 1 a) aufgearbeitet. Nach Fällung des Rohproduktes mit Ammoniak in Isopropanol/Diethylether (1:10) und Umkristallisation werden 4,1 g der Titelverbindung erhalten, Fp. 82—85° (Zers.).

b) Analog Beispiel 13 b) werden 9,6 g 6-Methyl-1,2,3,4-tetrahydrochinolin-2-on mit TEBA und Dimethylsulfat in Methylenchlorid/50% Natronlauge umgesetzt. Nach Extraktion mit Methylenchlorid erhält man 10,2 g 1,6 Dimethyl-1,2,3,4-tetrahydrochinolin-2-on als zähes Öl (98% d.Th.). Das Öl wird analog Beispiel 3 b) in wäßrig-basischer Lösung hydrolysiert.

Beispiel 15

4-[2-(N-Methyl-2,4-dichlorbenzamido)-phenyl]-buttersäure

$R^3$ = —$CH_3$, $R^4$ = $R^5$ = —Cl, $R^1$ = $R^2$ = $R^6$ = —H, n = 3

6,3 g 4-(2-N-Methylamino-phenyl)-buttersäure in wäßrig-basischer Lösung, pH 8—9, [hergestellt analog Beispiel 4 b)] werden mit 30 g festem Natriumhydrogencarbonat versetzt. Unter Rühren tropft man dazu eine Lösung von 7,5 g 2,4-Dichlorbenzoylchlorid in 50 ml Diethylether. 2 Stunden nach Zugabe wird analog Beispiel 4 a) aufgearbeitet. Man erhält nach Umkristallisation aus Essigsäureethylester/Petrolether (1:4) 1,6 g der Titelverbindung, Fp. 102—103°.

Beispiel 16

4-[2-(N-Methyl-3,4-dichlorbenzamido)-phenyl]-buttersäure

$R^3$ = —$CH_3$, $R^4$ = $R^5$ = —Cl, $R^1$ = $R^2$ = $R^6$ = —H, n = 3

Eine nach Beispiel 4 b) hergestellte wäßrig-basische Lösung von 5,4 g 4-(2-N-Methylamino-phenyl)-buttersäure wird bei pH 8,5 [analog Beispiel 4 a)] mit 5,2 g 3,4-Dichlorbenzoylchlorid in 50 ml Methylenchlorid versetzt. Nach Aufarbeitung und Umkristallisation aus Essigsäureethylester/Petrolether (1:1) isoliert man 5,7 g (63% d.Th.) der Titelverbindung, Fp. 120—121°.

Beispiel 17

4-[2-(N-Methyl-2-phenylbenzamido)-phenyl]-buttersäure

$R^3$ = —$CH_3$, $R^4$ = Phenyl, $R^1$ = $R^2$ = $R^5$ = $R^6$ = —H, n = 3

6,3 g 4-[2-N-Methylamino-phenyl)-buttersäure in wäßrig-basischer Lösung [s. Beispiel 4 b)]

werden mit 7,8 g Biphenyl-2-carbonsäurechlorid umgesetzt. Aufarbeitung und Umkristallisation aus Essigsäureethylester ergeben 4 g (30% d.Th.) der Titelverbindung, Fp. 154—155°.

Beispiel 18

4-[4-Methoxy-2-(N-methyl-2,4-dichlorbenzamido)-phenyl]-buttersäure

$$R^1 = OCH_3,\ R^3 = -CH_3,\ R^4 = R^5 = -Cl,\ R^2 = R^6 = -H,\ n = 3$$

a) Die gemäß b) erhaltene wäßrig-basische Lösung von 4-(4-Methoxy-2-N-methylamino-phenyl)-buttersäure wird analog Beispiel 1 a) mit 6,7 g 2,4-Dichlorbenzoylchlorid umgesetzt und aufgearbeitet. Umkristallisation des Rohproduktes aus Essigsäureethylester/Petrolether (1:1) liefert 4,8 g (38% d.Th.) der Titelverbindung, Fp. 90—91°.

b) Schmidt-Reaktion: 4 g Natriumazid werden portionsweise zu 8,3 g 7-Methoxy-1-tetralon in 35 ml Eisessig zugegeben. Anschließend tropft man 17 ml konzentrierte Schwefelsäure zu, ohne daß die Temperatur 40° übersteigt; dann wird auf Eis gegossen und mit 2 N Natronlauge neutralisiert. Die sich abscheidende Kristallmasse wird abgetrennt und aus Essigsäureethylester umkristallisiert. Man erhält 6,4 g 8-Methoxy-2,3,4,5-tetrahydro-1H-1-benzazepin-2-on (Fp. 137—138°).

Methylierung: Das durch Schmidt-Reaktion erhaltene Benzazepinon wird analog Beispiel 3 b) mit Natriumhydrid und Dimethylsulfat N-methyliert. Nach Aufarbeitung ergeben sich 6,8 g 8-Methoxy-1-methyl-2,3,4,5-tetrahydro-1-H-1-benzazepin-2-on.

Hydrolyse: Das durch N-Methylierung erhaltene Methylbenzazepinon wird gemäß Beispiel 1 b) mit überschüssigem Kaliumhydroxid in Ethylenglykolmonoethylether hydrolysiert, aufgearbeitet und als wäßrig-basische Lösung (pH 8,5) in a) eingesetzt.

Beispiel 19

Analog Beispiel 18 werden ausgehend von 7-Methoxy-1-tetralon, 6-Methoxy-1-tetralon und 5-Methoxy-1-tetralon durch Schmidt-Reaktion (zu den entsprechenden Methoxy-2,3,4,5-tetrahydro-1H-1-benzazepin-2-onen) mit anschließender Methylierung und Hydrolyse 4-(4-Methoxy-2-N-methylamino-phenyl)-buttersäure, 4-(5-Methoxy-2-N-methylamino-phenyl)-buttersäure und 4-(6-Methoxy-2-N-methylamino-phenyl)-buttersäure hergestellt und in ihren wäßrig-basischen Lösungen mit entsprechenden Benzoylchloriden zu folgenden erfindungsgemäßen Verbindungen acyliert:

a) 4-[2-(N-Methyl-4-chlorbenzamido)-4-methoxyphenyl]-buttersäure
Fp. 107—108° (aus Diethylether/Petrolether),

$$R^1 = OCH_3,\ R^3 = -CH_3,\ R^4 = -Cl,\ R^2 = R^5 = R^6 = -H,\ n = 3$$

b) 4-[2-(N-Methyl-3,4-dichlorbenzamido)-5-methoxyphenyl]-buttersäure
Fp. 87—88°

$$R^1 = OCH_3,\ R^3 = -CH_3,\ R^4 = R^5 = -Cl,\ R^2 = R^6 = -H,\ n = 3$$

c) 4-[2-(N-Methyl-2,4-dichlorbenzamido)-5-methoxyphenyl]-buttersäure
Öl, Natrium-Salz: Fp. 160° (Zers.)

$$R^1 = -OCH_3,\ R^3 = -CH_3,\ R^4 = R^5 = -Cl,\ R^2 = R^6 = -H,\ n = 3$$

d) 4-[2-(N-Methyl-4-chlorbenzamido)-6-methoxyphenyl]-buttersäure
Fp. 121—122°

$$R^1 = -OCH_3,\ R^3 = -CH_3,\ R^4 = -Cl,\ R^2 = R^5 = R^6 = -H,\ n = 3$$

Beispiel 20

5-[2-(N-Methyl-3-trifluormethylbenzamido)-phenyl]-valeriansäure

$$R^3 = -CH_3,\ R^4 = -CF_3,\ R^1 = R^2 = R^5 = -H,\ n = 4$$

a) Die nach b) erhaltene Lösung wird analog Beispiel 1 a) mit 6,9 g 3-Trifluormethylbenzoylchlorid umgesetzt, wobei die Einhaltung eines schwach basischen Milieus durch einen Überschuß an Natriumhydrogencarbonat (23 g) gewährleistet wird. Nach Aufarbeitung resultieren 8,8 g der Titelverbindung, Fp. 81—83° (68% d.Th.).

b) Analog Beispiel 18 b) werden 7,5 g Benzo-[b]-suberon nach Schmidt zum Lactam ringerweitert und anschließend am Stickstoff methyliert; man erhält 6,2 g 2-Aza-2-methylbenzo[c]-cyclooctanon als Öl (Gesamtausbeute 73% d.Th.). Dieses wird in Ethylenglykolmonoethylether mit Natriumhydroxid zu 5-(2-N-Methylaminophenyl)-valeriansäure hydrolysiert; die wäßrig-basische Lösung wird in a) eingesetzt.

Beispiel 21

5-[2-(N-Methyl-3,4-dichlorbenzamido)-phenyl]-valeriansäure

$R^3$ = —$CH_3$, $R^4$ = $R^5$ = —Cl, $R^1$ = $R^2$ = $R^6$ = —H, n = 4

Eine nach Beispiel 20 b) hergestellte Lösung von 5-(2-Methylaminophenyl)-valeriansäure wird analog Beispiel 1 a) mit 6,9 g 3,4-Dichlorbenzoylchlorid umgesetzt. Nach Aufarbeitung und Umkristallisation erhält man 3,7 g der Titelverbindung, Fp. 86—87°.

Beispiel 22

5-[2-(N-Methyl-4-chlorbenzamido)-phenyl]-valeriansäure

$R^3$ = —$CH_3$, R4 = —Cl, $R^1$ = $R^2$ = $R^5$ = $R^6$ = —H, n = 4

a) 5,4 g Diethyl-3-[2-(N-methyl-4-chlorbenzamido)-phenyl]-propylmalonat werden in 50 ml Toluol gelöst und mit 2,5 g Kaliumhydroxid in 25 ml Methanol versetzt. Nach 4 Tagen Rühren bei Raumtemperatur wird die freie 3-[2-(N-Methyl-4-chlorbenzamido)-phenyl]-propylmalonsäure durch Extraktion mit Chloroform als Öl isoliert; dieses spaltet bei Erhitzen auf 170° Kohlendioxid ab. Der Rückstand wird chromatographisch gereinigt und ergibt nach Umkristallisation aus Essigsäureethylester/Petrolether (1:1) 1,5 g (36% d.Th.) der Titelverbindung, Fp. 117—118°.

b) 15,8 g 3-[2-(N-Methyl-4-chlorbenzamido)-phenyl]-propionsäure werden mit 15 g Oxalylchlorid in Toluol 2 Stunden gerührt und anschließend im Vakuum eingedampft. Der Rückstand wird in 60 ml Dioxan aufgenommen und zu 1,6 g Natriumborhydrid in 40 ml Dioxan getropft. Danach wird 4 Stunden auf 100° erhitzt und anschließend im Eisbad 2 N Salzsäure bis zum Abklingen der Gasentwicklung zugegeben. Extraktive Aufarbeitung mit Diethylether ergibt 11,9 g 3-[2-(N-Methyl-4-chlorbenzamido)-phenyl]-propanol als Öl, das ohne Reinigung in 80 ml Toluol mit 3 g Pyridin und 6 g Tosylchlorid zum 3-[2-(N-Methyl-4-chlorbenzamido)phenyl]-propyltosylat umgesetzt wird (33 Stunden Rühren bei Raumtemperatur); die Ausbeute nach chromatographischer Reinigung an Silicagel mit Methylenchlorid beträgt 6,1 g (31% d.Th.). Dieses Tosylat wird in 70 ml Ethanol zu einer Lösung von 0,35 g Natrium in 60 ml Ethanol und 3,2 g Diethylmalonat getropft; anschließend wird 24 Stunden auf Rückfluß erhitzt. Aufarbeitung durch Extraktion mit Methylenchlorid und chromatographische Reinigung über Kieselgel mit Chloroform ergeben 5,4 g Diethyl-3-[2-N-methyl-4-chlorbenzamido)-phenyl]-propylmalonat (89% d.Th.) als Öl, das nach a) weiterverarbeitet wird.

Beispiel 23

6-[2-(N-Methyl-3-trifluormethylbenzamido)-phenyl]-capronsäure

$R^3$ = —$CH_3$, $R^4$ = —$CF_3$, $R^1$ = $R^2$ = $R^5$ = $R^6$ = —H, n = 5

4,1 g 4-[2-(N-Methyl-3-trifluorbenzamido)-phenyl]-n-butylmalonsäurediethylester [hergestellt analog Beispiel 22 b) aus 4-[2-(N-Methyl-3-trifluormethylbenzamido)-phenyl]-buttersäure über das entsprechende Butanol und Butyltosylat und dessen Umsetzung mit Diethylmalonat] werden analog Beispiel 22 a) mit Kaliumhydroxid in Toluol/Methanol verseift und ohne Reinigung durch Erhitzen decarboxyliert. Nach Umkristallisation aus Essigsäureethylester/Diethylether erhält man 1,9 g der Titelverbindung, Fp. 103—104°.

Beispiel 24

Ampullen mit 600 mg 3-[2-(N-Methyl-4-chlorbenzamido)-phenyl]-propionsäure, Chargengröße 250 kg, werden wie folgt hergestellt:

Es werden 25 kg 1,2-Propylenglykol und 150 kg aqua bidestillata vorgelegt, 15 kg 3-[2-(N-Methyl-4-chlorbenzamido)-phenyl]-propionsäure zugegeben und anschließend langsam, unter Rühren, ca. 19 kg Natronlauge (10 Gew.-% NaOH) zugegeben. Wenn sich alles gelöst hat, wird mit verdünnter Salzsäure der pH auf 7,5—8,0 eingestellt. 0,0625 kg Natriumpyrosulfit werden zugegeben, und die Mischung wird gerührt, bis sich alles gelöst hat. Mit aqua bidestillata wird auf 250 kg aufgefüllt. Die Lösung wird in 10 ml-Ampullen abgefüllt und bei 120° 30 Minuten im Autoklaven sterilisiert.

Beispiel 25

Ampullen mit 600 mg 4-[2-(N-Methyl-4-chlorbenzamido)-phenyl]-buttersäure, Chargengröße 250 kg, werden wie folgt hergestellt:

50 kg 1,2-Propylenglykol und 150 kg aqua bidestillata werden vorgelegt. Dann werden unter Rühren 15 kg 4-[2-(N-Methyl-4-chlorbenzamido)-phenyl]-buttersäure zugegeben. Anschließend werden ca. 18,5 kg 10-%ige Natronlauge hinzugegeben, und dann wird die Lösung mit verdünnter Salzsäure auf einen pH von 8,0 eingestellt. Mit aqua bidestillata wird auf 250 kg ergänzt. Die Lösung wird in 10 ml-Ampullen abgefüllt und 30 Minuten bei 120° im Autoklaven sterilisiert.

Beispiel 26

Tabletten mit 50 mg 3-[2-(N-Methyl-benzamido)-phenyl]-propionsäure werden wie folgt hergestellt:

25 kg 3-[2-(N-Methyl-benzamido)-phenyl]-propionsäure, 25 kg Xylit und 26 kg Calciumphosphat werden mit 2,5 kg Polyvinylpyrrolidon (MG~25 000; MG = Molekulargewicht) in ungefähr 6 l Wasser granuliert. Das Granulat wird durch ein Sieb von 1,25 mm Maschenweite gesiebt und nach dem Trocknen werden 8 kg Carboxymethylcellulose, 2,5 kg Talkum und 1 kg Magnesiumstearat zugegeben. Man verpreßt das trockene Granulat zu Tabletten von 8 mm Durchmesser, 250 mg Gewicht und einer Härte von 5—6 kg.

Beispiel 27

Tabletten mit 100 mg 4-[2-(N-Methyl-3,4-dichlorbenzamido)-phenyl]-buttersäure werden wie folgt hergestellt:

40 kg 4-[2-(N-Methyl-3,4-dichlorbenzamido)-phenyl]-buttersäure, 24 kg Xylit und 16 kg Calciumphosphat werden mit 4 kg Polyvinylpyrrolidon (MG~25 000) in ungefähr 5,5 l Wasser granuliert und durch ein Sieb von 1,25 mm Maschenweite gepreßt. Nach dem Trocknen werden 10 kg Carboxymethylcellulose, 4 kg Talkum und 2 kg Magnesiumstearat zugegeben. Auf einer Exzentermaschine wird das Granulat zu Tabletten von 9 mm Durchmesser, 250 mg Gewicht und einer Härte von 4—5 kg verpreßt.

Beispiel 28

Tabletten mit 300 mg 3-[2-(N-Methyl-3,4-dichlor-benzamido)-5-chlorphenyl]-propionsäure werden wie folgt hergestellt:

60 kg 3-[2-(N-Methyl-3,4-dichlor-benzamido)-5-chlorphenyl]-propionsäure, 12 kg Xylit und 8 kg Calciumphosphat werden mit 4 kg Polyvinylpyrrolidon (MG~25 000) in ungefähr 6 l Wasser granuliert und durch ein Sieb von 1,25 mm Maschenweite gepreßt. Nach dem Trocknen werden 10 kg Carboxymethylcellulose, 4 kg Talkum und 2 kg Magnesiumstearat zugegeben. Auf einem Rundläufer wird das Granulat zu Tabletten von 11 mm Durchmesser, 500 mg Gewicht und einer Härte von 6—7 kg verpreßt.

Beispiel 29

10 000 Kapseln mit einem Wirkstoffgehalt von 50 mg 4-[2-(N-Methyl-4-chlorbenzamido)-phenyl]-buttersäure werden aus folgenden Bestandteilten hergestellt:

500 g 4-[2-(N-Methyl-4-chlorbenzamido)-phenyl]-buttersäure, 495 g mikrokristalline Cellulose und 5 g amorphe Kieselsäure werden gut gemischt und in Hartgelatinekapseln Größe 4 abgefüllt.

Beispiel 30

10 000 Kapseln mit einem Wirkstoffgehalt von 50 mg 4-[2-(N-Methyl-3-trifluormethyl-benzamido)-phenyl]-buttersäure werden aus folgenden Bestandteilen hergestellt:

500 g 4-[2-(N-Methyl-3-trifluormethylbenzamido)-phenyl]-buttersäure, 495 g mikrokristalline Cellulose und 5 g amorphe Kieselsäure werden gut gemischt und in Hartgelatinekapseln Größe 4 abgefüllt.

Pharmakologie

Die $\omega$-[2-(N-Niederalkyl-benzamido)-phenyl]-alkansäuren und ihre Salze senken den Blutglucosespiegel, wobei sie sich in ihrer chemischen Struktur und ihrer Wirkungsweise grundlegend von pankreaswirksamen, betacytotropen Substanzen (z.B. Sulfonylharnstoffen) durch ihrer extrapankreatische, insbesondere hepatische Wirkung unterscheiden und sich den extrapankreatisch wirkenden Handelspräparaten, z.B. Buformin und Phenformin, überlegen erweisen.

In der anschließenden Tabelle werden die untersuchten Verbindungen durch eine laufende Nummer gekennzeichnet, die wie folgt zuzuordnen ist:

| Lfd. Nr. | Name der Verbindung |
|---|---|
| 1 | Buformin |
| 2 | Phenformin |
| 3 | 4-[2'-(N-Methyl-benzamido)-phenyl]-buttersäure |
| 4 | 3-[2-(N-Methyl-4-chlorbenzamido)-phenyl]-propionsäure |
| 5 | 3-[2-(N-Methyl-3,4-dichlor-benzamido)-phenyl]-propionsäure |
| 6 | 3-[2-(N-Methyl-benzamido)-phenyl]-propionsäure |
| 7 | 3-[5-Chlor-2-(N-methyl-3,4-dichlorbenzamido)-phenyl]-propionsäure |
| 8 | 3-[5-Chlor-2-(N-methyl-3-trifluormethylbenzamido)-phenyl]-propionsäure |
| 9 | 4-[2-(N-Methyl-4-chlor-benzamido)-phenyl]-buttersäure |
| 10 | 4-[2-(N-Methyl-3,4-dichlorbenzamido)-phenyl]-buttersäure |
| 11 | 4-[2-(N-Methyl-2,4-dichlorbenzamido)-phenyl]-buttersäure |
| 12 | 4-[2-(N-Methyl-3-trifluormethylbenzamido)-phenyl]-buttersäure |
| 13 | 4-[6-Methoxy-2-(N-methyl-4-chlorbenzamido)-phenyl]-buttersäure |
| 14 | 4-[5-Methoxy-2-(N-Methyl-3,4-dichlorbenzamido)-phenyl]-buttersäure |
| 15 | 5-[2-(N-Methyl-3,4-dichlorbenzamido)-phenyl]-valeriansäure |

In Tabelle I werden Untersuchungen des Einflusses von Vertreten der erfindungsgemäßen Verbindungen auf die Blutglucosekonzentration nüchterner stoffwechselgesunder Ratten nach einmaliger oraler Substanzgabe von 0,6 bis 1,0 mmol/kg Köpergewicht innerhalb von 24 Stunden wiedergegeben. Spalte A gibt jeweils die maximale Senkung in % in Bezug auf die Kontrollgruppe innerhalb von 24 Stunden nach Applikation der Verbindungen an. Gleichzeitig wird in Spalte B die Wirkstoffdosis (mg/kg) angegeben, die bei 50% der Tiere eine Senkung der Blutglucosekonzentration um mindestens 15% im Vergleich zur Kontrollgruppe bewirkt.

In der Spalte C werden Daten zur akuten Toxizität ($LD_{50}$ Maus p.o.) wiedergegeben.

Die Werte in Spalte D stellen die Konzentration ($ID_{50}$ mmol/l) der Substanzen im Perfusat dar, die an der isoliert perfundierten Rattenleber eine 50%ige Hemmung der Glucosebildung aus Lactat und Pyruvat bewirken.

| Tab. I Verb. Nr. | A Änderung der Blutglucosekonzentration in % | B $ED_{50}$ (mg /kg) | C $LD_{50}$ (mg /kg) | D $ID_{50}$ (mMol /kg) |
|---|---|---|---|---|
| 1 | –20 | 227 | 475 | unwirksam |
| 2 | – 2 | nicht bestimmbar | 410* | unwirksam |
| 3 | –27 | 205 | – | – |
| 4 | –16 | 426 | – | 0.140 |
| 5 | –11 | 363 | 1100 | 0.100 |
| 6 | –26 | 221 | 970 | >0.200 |
| 7 | –15 | – | >1000 | 0.034 |
| 8 | –26 | 185 | >1000 | 0.105 |
| 9 | –24 | 103 | >1000 | 0.125 |
| 10 | –17 | 198 | 250 (i.p.) | 0.035 |
| 11 | –17 | 245 | >1000 | 0.060 |
| 12 | –20 | 278 | 150 (i.p.) | 0.015 |
| 13 | –14 | >500 | – | 0.044 |
| 14 | –21 | – | – | 0.070 |
| 15 | – 5 | >700 | – | 0.050 |

* zitiert nach D. A. Blickens und S. J. Riggi, Toxicol. Appl. Pharmacol. *14* (1969) 393–400.

Zu Tabelle I:

A = Maximale Anderung der Blutglucosekonzentration (in %) in vivo im Vergleich zu Kontrolltieren

B = Dosis, die eine Senkung der Blutglucosekonzentration um 15 % bei 50 % der Tiere bewirkt

C = Toxizität

D = Dosis, die eine 50 %ige Hemmung der Glucosebildung aus Pyruvat und Lactat bewirkt

Die erfindungsgemäßen Verbindungen zeichnen sich gegenüber den Vergleichsverbindungen durch eine geringere Toxizität und teilweise durch eine stärkere und langanhaltende hypoglykämische Wirkung aus. Weiterhin sind sie durch eine wesentlich stärkere Hemmung der Glucosebildung aus Lactat und Pyruvat in der Leber gekennzeichnet. Während Buformin und Phenformin keine Hemmung bewirken, lassen sich mit den erfindungsgemäßen Verbindungen Hemmeffekte bis zu ca. 100% erzielen.

Die Ermittlung der pharmakologischen Eigenschaften erfolgte nach folgenden Methoden:

1. Blutglucosebestimmung nach einmaliger oraler Applikation

Zur Verwendung kommen junge männlich Sprague-Dawley-Ratten (Körpergewicht 160—200 g). Die Haltung der Tiere erfolgt Makrolon-Käfigen bis zu 5 Tieren pro Käfig (Raumtemperatur 23°C, rel. Luftfeuchtigkeit 55%, fester Tag/Nacht-Rythmus (12/12 h), Rattenstandarddiät Altromin[R]). Den Tieren wird 20 bis 22 Stunden vor der 1. Blutabnahme das Futter entzogen. Wasseraufnahme erfolgt ad libitum. Blutentnahmen erfolgen zu den Zeitpunkten 0, 2,6, und 24 Stunden durch Punktion aus dem retroorbitalen Plexus.

Nach Enteiweißung mit Perchlorsäure erfolgt die Blutglucosebestimmung mittels der enzymatischen HK/G—6—PDH-Methode nach R. Richterich (Klinische Chemie, Theorie und Praxis, 3. Aufl. 1971, S. Karger Verlag, Zürich-Basel, Seite 275). Zum Vergleich wird jeweils eine mit reinem Lösungsmittel behandelte Kontrollgruppe mituntersucht.

2. Hemmung der Glucosebildung an der isoliert perfundierten Rattenleber

Zur Verwendung kommen junge männliche Sprague-Dawley-Ratten (160—200 g). Die Haltung der Tiere erfolgt wie unter 1. beschrieben.

Den Tieren wird 20 bis 22 Stunden vor der Operation das Futter entzogen. Wasseraufnahme erfolgt ad libitum. Operation und Perfusion der Leber erfolgen nach der Technik von R. Scholz et al [Eur. J. Biochem. 38(1973)64—72]. Als Perfusionsflüssigkeit wird Krebs-Henseleit-Bicarbonat-Puffer (pH 7,4), der mit einer Sauerstoff/Kohlendioxid-Mischung (95/5) gesättigt ist und 1,6 mmol/l L-Lactate und 0,2 mmol/l Pyruvat enthält, verwandt. Die Perfusionsflüssigkeit wird über eine in die Pfortader eingeführte Kanüle in die Leber gepumpt. Die austretende Perfusionsflüssigkeit wird über eine in die Vena cava eingeführte Kanüle gesammelt und dann an einer Sauerstoffelektrode vorbeigeleitet. Die Leber wird ca. 2 Stunden perfundiert. Die Testverbindungen werden von der 32. bis 80. Minute der Perfusion in steigender Konzentration (0,01, 0,03, 0,10 mmol/l) infundiert.

Proben der Austrittsperfusionsflüssigkeit werden in Einminutenintervallen gesammelt und auf Glucose, Lactat und Pyruvat nach enzymatischen Standardmethoden analysiert. Der Sauerstoffgehalt wird kontinuierlich mittels einer Platin-Elektrode bestimmt. Die in Tabelle I angegebenen Hemmkonstanten ($ID_{50}$) beziehen sich auf den vor und nach Zugabe der Verbindungen vorliegenden Zustand, wobei die durch Lactat und Pyruvat allein bedingte Glucosebildung gleich 100% gesetzt wurde.

3. Bestimmung der Toxizität

Die Toxizitätsuntersuchungen werden an weiblichen NMRI-Mäusen (Körpergewicht 22—26 g) durchgeführt. Die Tiere (5 Tiere pro Dosis) erhalten 18 Stunden vor der Behandlung das Futter (Altromin®) auf 50g/50 Tiere reduziert und Wasser ad libitum. Verschiedene Dosen der Substanzen werden per os oder intraperitoneal verabreicht (Volumen 20 ml/kg). Die Beobachtungsdauer beträgt 7 Tage. Die $LD_{50}$, d.h. die Dosis, bei der 50% der Tiere sterben, wird aus der Dosiswirkungskurve graphisch ermittelt.

**Patentansprüche**

1. ω-[2-(N-Niederalkyl-benzamido)-phenyl]-alkansäuren der allgemeinen Formel I

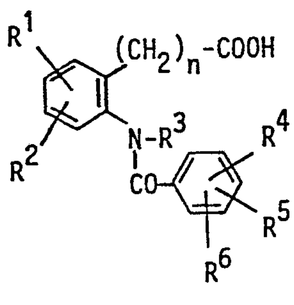

(I),

worin

n eine positive ganze Zahl von 2 bis 5 bedeutet,

$R^1$ ein Wasserstoffatom, ein Halogenatom, eine Niederalkylgruppe, eine Niederalkoxygruppe, eine Niederalkylmercaptogruppe, eine Trifluormethylgruppe oder eine gegebenenfalls durch ein Halogenatom oder eine Niederalkoxygruppe substituierte Phenylgruppe bedeutet,

$R^2$ ein Wasserstoffatom, ein Halogenatom, eine Niederalkylgruppe oder eine Niederalkoxygruppe bedeutet,

$R^3$ eine Niederalkylgruppe bedeutet,

$R^4$ ein Wasserstoffatom, ein Halogenatom, eine Hydroxygruppe, eine Niederalkylgruppe, eine Niederalkoxygruppe, eine Niederalkylmercaptogruppe, eine gegebenenfalls durch ein Halogenatom oder eine Nitrogruppe substituierte Phenylgruppe, eine Nitrogruppe, eine gegebenenfalls niederalkylierte Aminogruppe, eine Niederalkylcarbonylgruppe, eine gegebenenfalls durch ein Halogenatom substituierte Benzoylgruppe, eine Trifluormethylgruppe, eine Trifluormethoxygruppe oder eine Trifluormethylmercaptogruppe bedeutet,

$R^5$ ein Wasserstoffatom, ein Halogenatom, eine Niederalkylgruppe oder eine Niederalkoxygruppe bedeutet,

$R^6$ ein Wasserstoffatom, eine Niederalkylgruppe oder eine Niederalkoxygruppe bedeutet,

und ihre Salze mit anorganischen oder organischen Basen.

2. $\omega$-[2-(N-Niederalkyl-benzamido)-phenyl]-alkansäuren der allgemeinen Formel I*

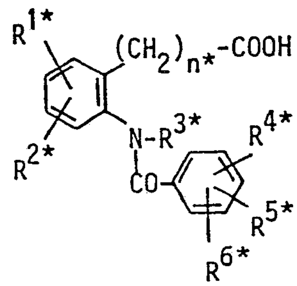

(I*),

worin

$n^*$ eine positive ganze Zahl von 2 bis 5 bedeutet,

$R^{1*}$ ein Wasserstoffatom, ein Chloratom, ein Bromatom, eine Niederalkylgruppe, eine Methoxygruppe oder eine Phenylgruppe bedeutet,

$R^{2*}$ ein Wasserstoffatom, ein Chloratom, ein Bromatom, eine Methylgruppe oder eine Methoxygruppe bedeutet,

$R^{3*}$ eine geradkettige Niederalkylgruppe bedeutet,

$R^{4*}$ ein Wasserstoffatom, ein Fluoratom, ein Chloratom, ein Bromatom, eine Niederalkylgruppe, eine Hydroxygruppe, eine Niederalkoxygruppe, eine Hydroxygruppe, eine Niederalkoxygruppe, eine Phenylgruppe, eine Nitrogruppe, eine Aminogruppe, eine Dimethylaminogruppe oder eine Trifluormethylgruppe bedeutet,

$R^{5*}$ ein Wasserstoffatom, ein Chloratom, eine Niederalkylgruppe oder eine Niederalkoxygruppe bedeutet,

$R^{6*}$ ein Wasserstoffatom, eine Niederalkylgruppe oder eine Niederalkoxygruppe bedeutet,

und ihre Salze mit anorganischen oder organischen Basen.

3. $\omega$-[2-(N-Niederalkyl-benzamido)-phenyl]-alkansäuren der allgemeinen Formel I**

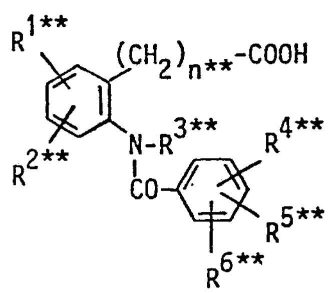

(I**),

worin

$n^{**}$ eine positive ganze Zahl von 2 bis 5 bedeutet,

$R^{1**}$ ein Wasserstoffatom, eine Methylgruppe, eine Methoxygruppe, ein Chloratom, ein Bromatom oder eine Phenylgruppe bedeutet,

$R^{2**}$ ein Wasserstoffatom, ein Chloratom oder eine Methylgruppe bedeutet,

$R^{3**}$ eine geradkettige Alkylgruppe mit 1 bis 4 Kohlenstoffatomen bedeutet,

$R^{4**}$ ein Wasserstoffatom, ein Chloratom, ein Bromatom, eine Methylgruppe, eine Niederalkoxygruppe, eine Phenylgruppe oder eine Trifluormethylgruppe bedeutet,

$R^{5**}$ ein Wasserstoffatom, ein Chloratom, eine Niederalkylgruppe oder eine Methoxygruppe bedeutet,

$R^{6**}$ ein Wasserstoffatom, eine Niederalkylgruppe oder eine Methoxygruppe bedeutet,

und ihre Salze mit anorganischen oder organischen Basen.

4. $\omega$-[2-(N-Niederalkyl-benzamido)-phenyl]-alkansäuren der allgemeinen Formel I***

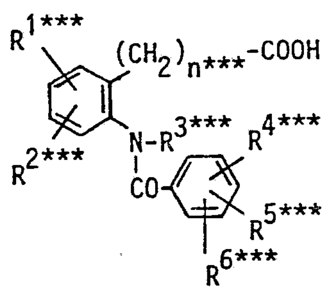

(I***),

worin

$n^{***}$ 2 oder 3 bedeutet,

$R^{1***}$ ein Wasserstoffatom, ein Chloratom, eine Methylgruppe oder eine Methoxygruppe bedeutet,

$R^{2***}$ ein Wasserstoffatom oder eine Methylgruppe bedeutet,

$R^{3***}$ eine Methylgruppe oder eine Ethylgruppe bedeutet,
$R^{4***}$ ein Wasserstoffatom, ein Chloratom, eine Phenylgruppe oder eine Trifluormethylgruppe bedeutet,
$R^{5***}$ ein Wasserstoffatom oder ein Chloratom bedeutet,
$R^{6***}$ ein Wasserstoffatom bedeutet,
und ihre pharmakologisch verträglichen Salze mit anorganischen oder organischen Basen.

5. $\omega$-[2-(N-Niederalkyl-benzamido)-phenyl]-alkansäuren der allgemeinen Formel I****

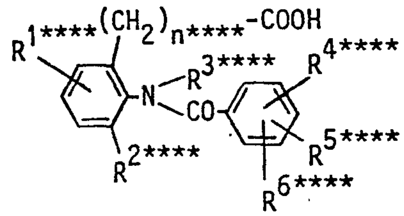

(I****),

worin
$n^{****}$ 2 oder 3 bedeutet,
$R^{1****}$ ein Wasserstoffatom, ein Chloratom, eine Methylgruppe oder eine Methoxygruppe bedeutet,
$R^{2****}$ ein Wasserstoffatom bedeutet,
$R^{3****}$ eine Methylgruppe bedeutet,
$R^{4****}$ ein Wasserstoffatom, ein Chloratom oder eine Trifluormethylgruppe bedeutet,
$R^{5****}$ ein Wasserstoffatom oder ein Chloratom bedeutet,
$R^{6****}$ ein Wasserstoffatom bedeutet,
und ihre pharmakologisch verträglichen Salze mit anorganischen oder organischen Basen.

6. Arzneimittel enthaltend eine oder mehrere Verbindungen gemäß Anspruch 1 bis 5.

7. Verfahren zur Herstellung von $\omega$-[2-(N-Niederalkyl)-benzamido)-phenyl]alkansäuren der allgemeinen Formel I

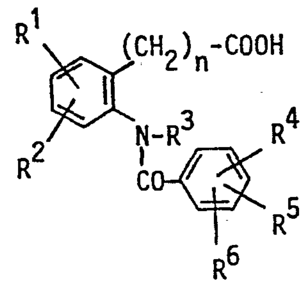

(I),

worin
n eine positive ganze Zahl von 2 bis 5 bedeutet,
$R^1$ ein Wasserstoffatom, ein Halogenatom, eine Niederalkylgruppe, eine Niederalkoxygruppe, eine Niederalkylmercaptogruppe, eine Trifluormethylgruppe oder eine gegebenenfalls durch ein Halogenatom oder eine Niederalkoxygruppe substituierte Phenylgruppe bedeutet,
$R^2$ ein Wasserstoffatom, ein Halogenatom, eine Niederalkylgruppe oder eine Niederalkoxygruppe bedeutet,
$R^3$ eine Niederalkylgruppe bedeutet,
$R^4$ ein Wasserstoffatom, ein Halogenatom, eine Hydroxygruppe, eine Niederalkylgruppe, eine Niederalkoxygruppe, eine Niederalkylmercaptogruppe, eine gegebenenfalls durch ein Halogenatom oder eine Nitrogruppe substituierte Phenylgruppe, eine Nitrogruppe, eine gegebenenfalls niederalkylierte Aminogruppe, eine Niederalkylcarbonylgruppe, eine gegebenenfalls durch ein Halogenatom substituierte Benzoylgruppe, eine Trifluormethylgruppe, eine Trifluormethoxygruppe oder eine Trifluormethylmercaptogruppe bedeutet,
$R^5$ ein Wasserstoffatom, ein Halogenatom, eine Niederalkylgruppe oder eine Niederalkoxygruppe bedeutet,
$R^6$ ein Wasserstoffatom, eine Niederalkylgruppe oder eine Niederalkoxygruppe bedeutet,
und ihrer Salze mit anorganischen oder organischen Basen, dadurch gekennzeichnet, daß man

a) eine $\omega$-(2-Niederalkylamino-phenyl)-alkansäure der allgemeinen Formel II

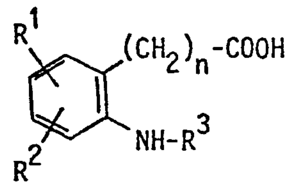

(II),

worin

n, $R^1$, $R^2$ und $R^3$ die oben angegebene Bedeutung haben, oder ein Salz davon mit einem Benzoylderivat der allgemeinen Formel III

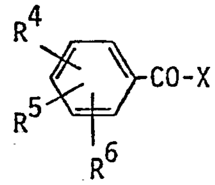

(III),

worin

X ein Halogenatom bedeutet und $R^4$, $R^5$ und $R^6$ die oben angegebene Bedeutung haben, in basischer Lösung acyliert und gewünschtenfalls anschließend erhaltene freie Säuren in ihre Salze oder erhaltene Salze in die freie Säure überführt oder

b) eine ω-[2-(N-Niederalkyl-benzamido)-phenyl]-alkylverbindung der allgemeinen Formel IV

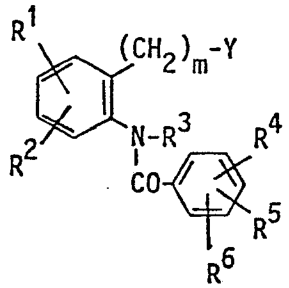

(IV),

worin

$R^1$, $R^2$, $R^3$, $R^4$, $R^5$ und $R^6$ die oben angegebene Bedeutung haben, m eine positive ganze Zahl von 1 bis 4 und Y eine Fluchtgruppe bedeuten, mit einem Dialkylmalonat, vorzugsweise einem Diniederalkylmalonat, in Gegenwart basischer Kondensationsmittel umsetzt und den erhaltenen substituierten Malonester verseift und decarboxyliert und gewünschtenfalls anschließend erhaltene freie Säuren in ihre Salze oder erhaltene Salze in die freien Säuren überführt oder

c) zur Herstellung von Verbindungen der Formel I, worin n = 2 ist, eine 2-(N-Niederalkyl-benzamido)-zimtsäure der allgemeinen Formel V

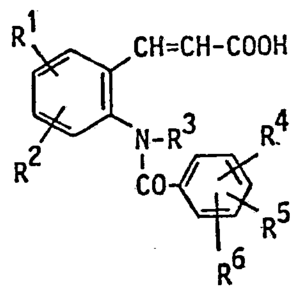

(V),

worin

$R^1$, $R^2$, $R^3$, $R^4$, $R^5$ und $R^6$ die oben angegebene Bedeutung haben, oder ein Salz oder einen Niederalkylester davon hydriert und gegebenenfalls anschließend erhaltene ω-[2-(N-Niederalkyl-benzamido)-phenyl]-propionsäureniederalkylester in die freien Säuren oder deren Salze überführt.

8. Verfahren zur Herstellung von ω-[2-(N-Niederalkyl-benzamido)-phenyl]-alkansäuren der allgemeinen Formel I*

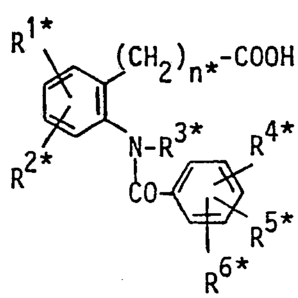

(I*),

worin

n* eine positive ganze Zahl von 2 bis 5 bedeutet,

$R^{1*}$ ein Wasserstoffatom, ein Chloratom, ein Bromatom, eine Niederalkylgruppe, eine Methoxygruppe oder eine Phenylgruppe bedeutet,

$R^{2*}$ ein Wasserstoffatom, ein Chloratom, ein Bromatom, eine Methylgruppe oder eine Methoxygruppe bedeutet,

$R^{3*}$ eine geradkettige Niederalkylgruppe bedeutet,

$R^{4*}$ ein Wasserstoffatom, ein Fluoratom, ein Chloratom, ein Bromatom, eine Niederalkylgruppe, eine Hydroxygruppe, eine Niederalkoxygruppe, eine Phenylgruppe, eine Nitrogruppe, eine Aminogruppe, eine Dimethylaminogruppe oder eine Trifluormethylgruppe bedeutet,

$R^{5*}$ ein Wasserstoffatom, ein Chloratom, eine Niederalkylgruppe oder eine Niederalkoxygruppe bedeutet,

$R^{6*}$ ein Wasserstoffatom, eine Niederalkylgruppe oder eine Niederalkoxygruppe bedeutet,

und ihrer Salze mit anorganischen oder organischen Basen, dadurch gekennzeichnet, daß man

a) eine ω-(2-Niederalkylamino-phenyl)-alkansäure der allgemeinen Formel II*

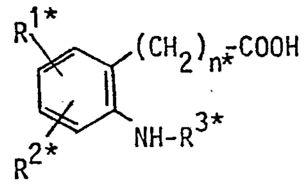

(II*),

worin

$n^*$, $R^{1*}$, $R^{2*}$ und $R^{3*}$ die oben angegebene Bedeutung haben, oder ein Salz davon mit einem Benzoylderivat der allgemeinen Formel III*

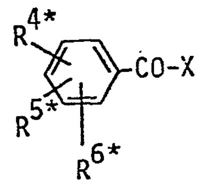

(III*),

worin

X ein Halogenatom bedeutet und $R^{4*}$, $R^{5*}$ und $R^{6*}$ die oben angegebene Bedeutung haben, in basischer Lösung acyliert und gewünschtenfalls anschließend erhaltene freie Säuren in ihre Salze oder erhaltene Salze in die freien Säuren überführt oder

b) eine ω-[2-(N-Niederalkyl-benzamido)-phenyl]-alkylverbindung der allgemeinen Formel IV*

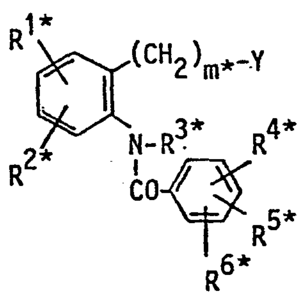

(IV*),

worin

$R^{1*}$, $R^{2*}$, $R^{3*}$, $R^{4*}$, $R^{5*}$ und $R^{6*}$ die oben angegebene Bedeutung haben, $m^*$ eine positive ganze Zahl von 1 bis 4 und Y eine Fluchtgruppe bedeuten, mit einem Dialkylmalonat, vorzugsweise einem Diniederalkylmalonat, in Gegenwart basischer Kondensationsmittel umsetzt und den erhaltenen substituierten Malonester verseift und decarboxyliert und gewünschtenfalls anschließend erhaltene freie Säuren in ihre Salz oder erhaltene Salze in die freien Säuren überführt oder

c) zur Herstellung von Verbindungen der Formel I*, worin $n^* = 2$ ist, eine 2-(N-Niederalkyl-benzamido)zimtsäure der allgemeinen Formel V*

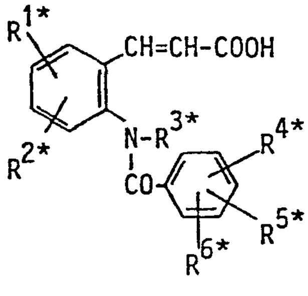

(V*),

worin

$R^{1*}$, $R^{2*}$, $R^{3*}$, $R^{4*}$, $R^{5*}$ und $R^{6*}$ die oben angegebene Bedeutung haben, oder ein Salz oder einen Niederalkylester davon hydriert und gegebenenfalls anschließend erhaltene ω-[2-(N-Niederalkyl-benzamido)-phenyl]-propionsäureniederalkylester in die freien Säuren oder deren Salze überführt.

9. Verfahren zur Herstellung von ω-[2-N-Niederalkyl-benzamido)-phenyl]-alkansäuren der allgemeinen Formel I**

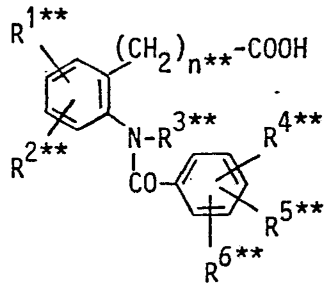

(I**),

worin

n** eine positive ganze Zahl von 2 bis 5 bedeutet,

$R^{1**}$ ein Wasserstoffatom, eine Methylgruppe, eine Methoxygruppe, ein Chloratom, ein Bromatom oder eine Phenylgruppe bedeutet,

$R^{2**}$ ein Wasserstoffatom, ein Chloratom oder eine Methylgruppe bedeutet,

$R^{3**}$ eine geradkettige Alkylgruppe mit 1 bis 4 Kohlenstoffatomen bedeutet,

$R^{4**}$ ein Wasserstoffatom, ein Chloratom, ein Bromatom, eine Methylgruppe, eine Niederalkoxygruppe, eine Phenylgruppe oder eine Trifluormethylgruppe bedeutet,

$R^{5**}$ ein Wasserstoffatom, ein Chloratom, eine Niederalkylgruppe oder eine Methoxygruppe bedeutet,

$R^{6**}$ ein Wasserstoffatom, eine Niederalkylgruppe oder eine Methoxygruppe bedeutet,

und ihrer Salze mit anorganischen oder organischen Basen, dadurch gekennzeichnet, daß man

a) eine ω-(2-Niederalkylamino-phenyl)-alkansäure der allgemeinen Formel II**

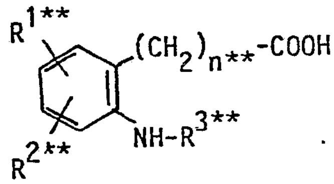

(II**),

worin

n**, $R^{1**}$, $R^{2**}$ und $R^{3**}$ die oben angegebene Bedeutung haben, oder ein Salz davon mit einem Benzoylderivat der allgemeinen Formel III**

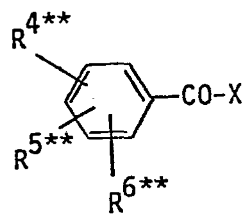

(III**),

worin

X ein Halogenatom bedeutet und $R^{4**}$, $R^{5**}$ und $R^{6**}$ die oben angegebene Bedeutung haben, in basischer Lösung acyliert und gewünschtenfalls anschließend erhaltene freie Säuren in ihre Salze oder erhaltene Salze in die freien Säuren überführt oder

b) eine ω-[2-(N-Niederalkyl-benzamido)-phenyl]-alkylverbindung der allgemeinen Formel IV**

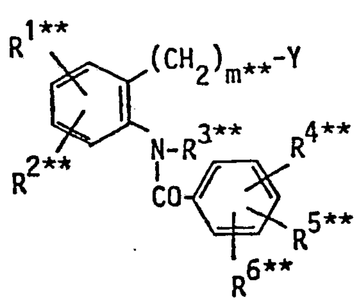

(IV**),

worin

$R^{1**}$, $R^{2**}$, $R^{3**}$, $R^{4**}$, $R^{5**}$ und $R^{6**}$ die oben angegebene Bedeutung haben, m** eine positive ganze Zahl von 1 bis 4 und Y eine Fluchtgruppe bedeuten, mit einem Dialkylmalonat, vorzugsweise

einem Diniederalkylmalonat, in Gegenwart basischer Kondensationsmittel umsetzt und den erhaltenen substituierten Malonester verseift und decarboxyliert und gewünschtenfalls anschließend erhaltene freie Säuren in ihre Salze oder erhaltene Salze in die freien Säuren überführt oder

c) zur Herstellung von Verbindungen der Formel I**, worin n** = 2 ist, eine 2-(N-Niederalkyl-benzamido)-zimtsäure der allgemeinen Formel V**

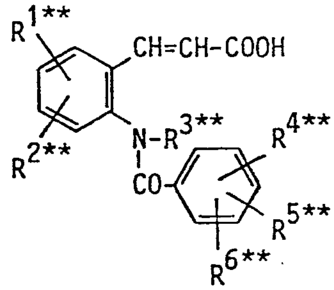

(V**),

worin

R^1**, R^2**, R^3**, R^4**, R^5** und R^6** die oben angegebene Bedeutung haben, oder ein Salz oder einen Niederalkylester davon hydriert und gegebenenfalls anschließend erhaltene $\omega$-[2-(N-Niederalkyl-benzamido)-phenyl]-propionsäureniederalkylester in die freie Säure oder deren Salze überführt.

10. Verfahren zur Herstellung von $\omega$-[2-(N-Niederalkyl-benzamido)-phenyl]-alkansäuren der allgemeinen Formel I***

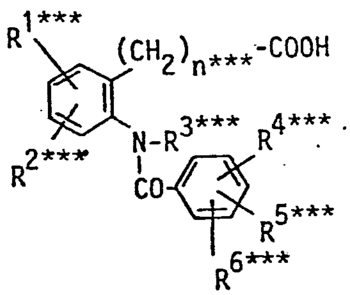

(I***),

worin

n*** 2 oder 3 bedeutet,

R^1*** ein Wasserstoffatom, ein Chloratom, eine Methylgruppe oder eine Methoxygruppe bedeutet,

R^2*** ein Wasserstoffatom oder eine Methylgruppe bedeutet,

R^3*** eine Methylgruppe oder eine Ethylgruppe bedeutet,

R^4*** ein Wasserstoffatom, ein Chloratom, eine Phenylgruppe oder eine Trifluormethylgruppe bedeutet,

R^5*** ein Wasserstoffatom oder ein Chloratom bedeutet,

R^6*** ein Wasserstoffatom bedeutet,

und ihrer pharmakologisch verträglichen Salze mit anorganischen oder organischen Basen, dadurch gekennzeichnet, daß man

a) eine $\omega$-(2-Niederalkylamino-phenyl)-alkansäure der allgemeinen Formel II***

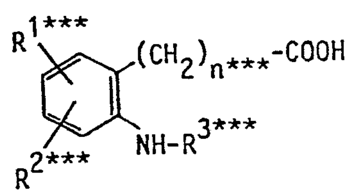

(II*** ),

worin

n***, R^1***, R^2*** und R^3*** die oben angegebene Bedeutung haben, oder ein Salz davon mit einem Benzoylderivat der allgemeinen Formel III***

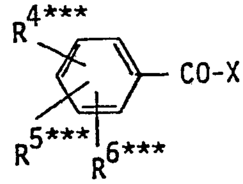

(III***),

worin

X ein Halogenatom bedeutet und R^4***, R^5*** und R^6*** die oben angegebene Bedeutung haben, in basischer Lösung acyliert und gewünschtenfalls anschließend erhaltene freie Säuren in ihre Salze oder erhaltene Salze in die freien Säuren überführt oder

b) eine $\omega$-[2-(N-Niederalkyl-benzamido)-phenyl]-alkylverbindung der allgemeinen Formel IV***

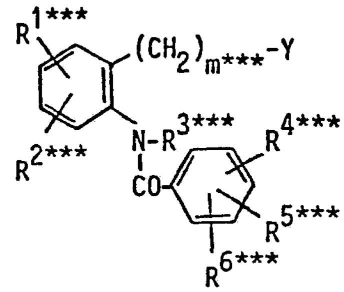

(IV***),

worin

R1***, R2***, R3***, R4***, R5*** und R6*** die oben angegebene Bedeutung haben, m*** 1 oder 2 und Y eine Fluchtgruppe bedeuten, mit einem Dialkylmalonat, vorzugsweise einem Diniederalkylmalonat, in Gegenwart basischer Kondensationsmittel umsetzt und den erhaltenen substituierten Malonester verseift und decarboxyliert und gewünschtenfalls anschließend erhaltene freie Säuren in ihre Salze oder erhaltene Salze in die freien Säuren überführt oder

c) zur Herstellung von Verbindungen der Formel I***, worin n*** = 2 ist, eine 2-(N-Niederalkyl-benzamido)-zimtsäure der allgemeinen Formel V***

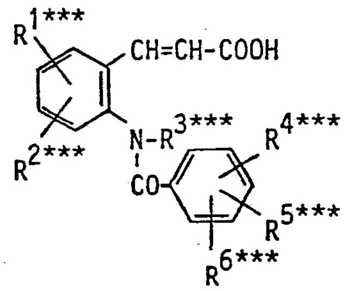

(V***),

worin

R1***, R2***, R3***, R4***, R5*** und R6*** die oben angegebene Bedeutung haben, oder ein Salz oder einen Niederalkylester davon hydriert und gegebenenfalls anschließend erhaltene $\omega$-[2-(N-Niederalkyl-benzamido)-phenyl]-propionsäureniederalkylester in die freie Säure oder deren Salze überführt.

11. Verfahren zur Herstellung von $\omega$-[2-(N-Niederalkyl-benzamido)-phenyl]-alkansäuren der allgemeinen Formel I****

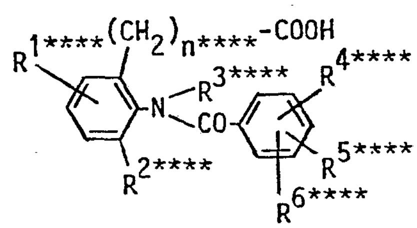

(I****),

worin

n**** 2 oder 3 bedeutet,

R1**** ein Wasserstoffatom, ein Chloratom, eine Methylgruppe oder eine Methoxygruppe bedeutet,

R2**** ein Wasserstoffatom bedeutet,

R3**** eine Methylgruppe bedeutet,

R4**** ein Wasserstoffatom, ein Chloratom oder eine Trifluormethylgruppe bedeutet,

R5**** ein Wasserstoffatom oder ein Chloratom bedeutet,

R6**** ein Wasserstoffatom bedeutet,

und ihrer pharmakologisch verträglichen Salze mit anorganischen oder organischen Basen, dadurch gekennzeichnet, daß man

a) eine $\omega$-(2-Niederalkylamino-phenyl)-alkansäure der allgemeinen Formel II****

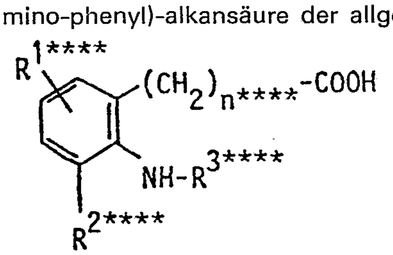

(II****),

worin

$n^{****}$, $R^{1****}$, $R^{2****}$ und $R^{3****}$ die oben angegebene Bedeutung haben, oder ein Salz davon mit einem Benzoylderivat der allgemeinen Formel III****

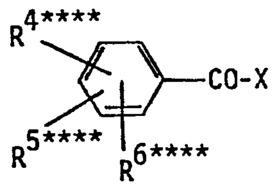

(III****),

worin

X ein Halogenatom bedeutet und $R^{4****}$, $R^{5****}$ und $R^{6****}$ die oben angegebene Bedeutung haben, in basischer Lösung acyliert und gewünschtenfalls anschließend erhaltene freie Säuren in ihre Salze oder erhaltene Salze in die freien Säuren überführt oder

b) eine ω-[2-(N-Niederalkyl-benzamido)-phenyl]-alkylverbindung der allgemeinen Formel IV****

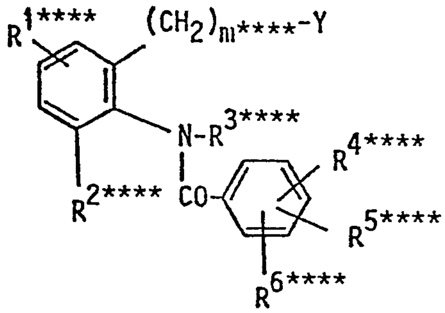

(IV****),

worin

$R^{1****}$, $R^{2****}$, $R^{3****}$, $R^{4****}$, $R^{5****}$ und $R^{6****}$ die oben angegebene Bedeutung haben, $m^{****}$ 1 oder 2 und Y eine Fluchtgruppe bedeuten, mit einem Dialkylmalonat, vorzugsweise einem Diniederalkylmalonat, in Gegenwart basischer Kondensationsmittel umsetzt und den erhaltenen substituierten Malonester verseift und decarboxyliert und gewünschtenfalls anschließend erhaltene freie Säuren in ihre Salze oder erhaltene Salze in die freien Säuren überführt oder

c) zur Herstellung von Verbindungen der Formel I****, worin $n^{****} = 2$ ist, eine 2-(N-Niederalkyl-benzamido)-zimtsäure der allgemeinen Formel V****

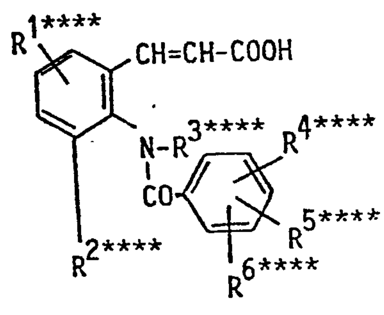

(V****),

worin $R^{1****}$, $R^{2****}$, $R^{3****}$, $R^{4****}$, $R^{5****}$ und $R^{6****}$ die oben angegebene Bedeutung haben, oder ein Salz oder einen Niederalkylester davon hydriert und gegebenenfalls anschließend erhaltene ω-[2-(N-Niederalkyl-benzamido)-phenyl]-propionsäureniederalkylester in die freie Säure oder deren Salze überführt.

12. Verbindungen nach Anspruch 1 bis 5 zur Anwendung bei der Bekämpfung und Prophylaxe von Krankheiten, die auf Störungen des Glucosestoffwechsel beruhen.

13. Verwendung von Verbindungen nach Anspruch 1 bis 5 zur Herstellung von Arzneimitteln, die zur Bekämpfung und Prophylaxe von Krankheiten, die auf Störungen des Glucosestoffwechsels beruhen, eingesetzt werden.

## Claims

1. ω-[2-(N-lower alkyl-benzamido)-phenyl]-alkanoic acids of the general formula I

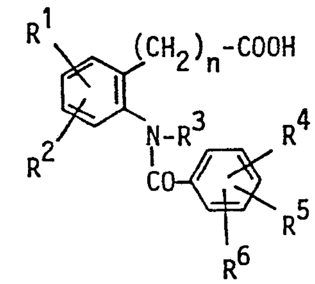

(I),

wherein
n denotes a positive whole number from 2 to 5,
$R^1$ denotes a hydrogen atom, a halogen atom, a lower alkyl group, a lower alkoxy group, a lower alkylmercapto group, a trifluoromethyl group or a phenyl group which may be substituted by a halogen atom or a lower alkoxy group,
$R^2$ denotes a hydrogen atom, a halogen atom, a lower alkyl group or a lower alkoxy group,
$R^3$ denotes a lower alkyl group,
$R^4$ denotes a hydrogen atom, a halogen atom, a hydroxyl group, a lower alkyl group, a lower alkoxy group, a lower alkylmercapto group, a phenyl group which may be substituted by a halogen atom or a nitro group, a nitro group, an amino group which may be lower alkylated, a lower alkylcarbonyl group, a benzoyl group which may be substituted by a halogen atom, a trifluoromethyl group, a trifluoromethoxy group or a trifluoromethylmercapto group,
$R^5$ denotes a hydrogen atom, a halogen atom, a lower alkyl group or a lower alkoxy group,
$R^6$ denotes a hydrogen atom, a lower alkyl group or a lower alkoxy group,
and their salts with inorganic or organic bases.

2. ω-[2-(N-lower alkyl-benzamido)-phenyl]-alkanoic acids of the general formula I*

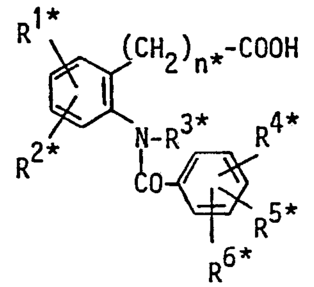

(I*),

wherein
n* denotes a positive whole number from 2 to 5,
$R^{1*}$ denotes a hydrogen atom, a chlorine atom, a bromine atom, a lower alkyl group, a methoxy group or a phenyl group,
$R^{2*}$ denotes a hydrogen atom, a chlorine atom, a bromine atom, a methyl group or a methoxy group,
$R^{3*}$ denotes a straight-chain lower alkyl group,
$R^{4*}$ denotes a hydrogen atom, a fluorine atom, a chlorine atom, a bromine atom, a lower alkyl group, a hydroxyl group, a lower alkoxy group, a phenyl group, a nitro group, an amino group, a dimethylamino group or a trifluoromethyl group,
$R^{5*}$ denotes a hydrogen atom, a chlorine atom, a lower alkyl group or a lower alkoxy group,
$R^{6*}$ denotes a hydrogen atom, a lower alkyl group or a lower alkoxy group,
and their salts with inorganic or organic bases.

3. ω-[2-(N-lower alkyl-benzamido)-phenyl]-alkanoic acids of the general formula I**

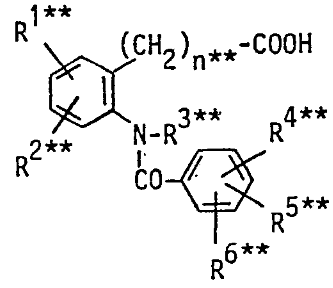

(I**),

wherein

n** denotes a positive whole number from 2 to 5,
R1** denotes a hydrogen atom, a methyl group, a methoxy group, a chlorine atom, a bromine atom or a phenyl group,
R2** denotes a hydrogen atom, a chlorine atom or a methyl group,
R3** denotes a straight-chain alkyl group with 1 to 4 carbon atoms,
R4** denotes a hydrogen atom, a chlorine atom, a bromine atom, a methyl group, a lower alkoxy group, a phenyl group or a trifluoromethyl group,
R5** denotes a hydrogen atom, a chlorine atom, a lower alkyl group or a methoxy group,
R6** denotes a hydrogen atom, a lower alkyl group or a methoxy group, and their salts with inorganic or organic bases.

4. ω-[2-(N-lower alkyl-benzamido)-phenyl]-alkanoic acids of the general formula I***

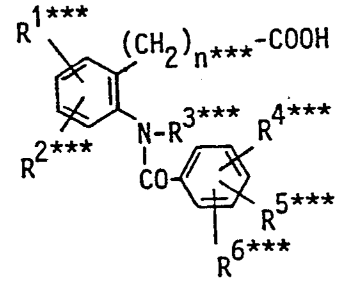

(I***),

wherein
n*** denotes 2 or 3,
R1*** denotes a hydrogen atom, a chlorine atom, a methyl group or a methoxy group,
R2*** denotes a hydrogen atom or a methyl group,
R3*** denotes a methyl group or an ethyl group,
R4*** denotes a hydrogen atom, a chlorine atom, a phenyl group or a trifluoromethyl group,
R5*** denotes a hydrogen atom or a chlorine atom,
R6*** denotes a hydrogen atom, and their pharmacologically compatible salts with inorganic or organic bases.

5. ω-[2-(N-lower alkyl-benzamido)-phenyl]-alkanoic acids of the general formula I****

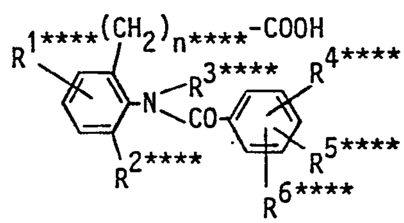

(I****),

wherein
n**** denotes 2 or 3,
R1**** denotes a hydrogen atom, a chlorine atom, a methyl group or a methoxy group,
R2**** denotes a hydrogen atom,
R3**** denotes a methyl group,
R4**** denotes a hydrogen atom, a chlorine atom or a trifluoromethyl group,
R5**** denotes a hydrogen atom or a chlorine atom,
R6**** denotes a hydrogen atom, and their pharmacologically compatible salts with inorganic or organic bases.

6. Medicaments containing one or more compounds according to claims 1 to 5.

7. Process for the preparation of ω-[2-(N-lower alkyl-benzamido)-phenyl]-alkanoic acids of the general formula I

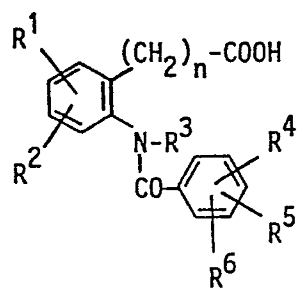

(I),

wherein
n denotes a positive whole number from 2 to 5,

$R^1$ denotes a hydrogen atom, a halogen atom, a lower alkyl group, a lower alkoxy group, a lower alkylmercapto group, a trifluoromethyl group or a phenyl group which may be substituted by a halogen atom or a lower alkoxy group,
$R^2$ denotes a hydrogen atom, a halogen atom, a lower alkyl group or a lower alkoxy group,
$R^3$ denotes a lower alkyl group,
$R^4$ denotes a hydrogen atom, a halogen atom, a hydroxyl group, a lower alkyl group, a lower alkoxy group, a lower alkylmercapto group, a phenyl group which may be substituted by a halogen atom or a nitro group, a nitro group, an amino group which may be lower alkylated, a lower alkylcarbonyl group, a benzoyl group which may be substituted by a halogen atom, a trifluoromethyl group, a trifluoromethoxy group or a trifluoromethylmercapto group,
$R^5$ denotes a hydrogen atom, a halogen atom, a lower alkyl group or a lower alkoxy group,
$R^6$ denotes a hydrogen atom, a lower alkyl group or a lower alkoxy group, and their salts with inorganic or organic bases, characterised in that

a) a $\omega$-(2-lower alkylamino-phenyl)-alkanoic acid of the general formula II

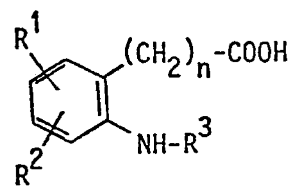

(II),

wherein

n, $R^1$, $R^2$ and $R^3$ have the meaning stated above, or a salt thereof, is acylated in basic solution with a benzoyl derivative of the general formula III

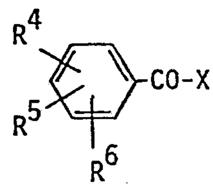

(III),

wherein

X denotes a halogen atom and $R^4$, $R^5$ and $R^6$ have the meaning stated above, and, if desired, obtained free acids are subsequently converted into their salts, or obtained salts are subsequently converted into the free acids, or

b) a $\omega$-[2-(N-lower alkyl-benzamido)-phenyl]-alkyl-compound of the general formula IV

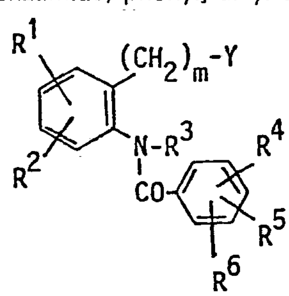

(IV),

wherein

$R^1$, $R^2$, $R^3$, $R^4$, $R^5$ and $R^6$ have the meaning stated above, m is a positive whole number from 1 to 4 and Y is a leaving group, is reacted with a dialkyl malonate, preferably with a di-lower-alkyl-malonate, in the presence of basic condensation agents, and the resulting substituted malonic ester is saponificated and decarboxylated and, if desired, obtained free acids are subsequently converted into their salts, or obtained salts are subsequently converted into the free acids,

or

c) in order to prepare compounds of the formula I, wherein n = 2, a 2-(N-lower alkyl-benzamido)-cinnamic acid of the general formula V

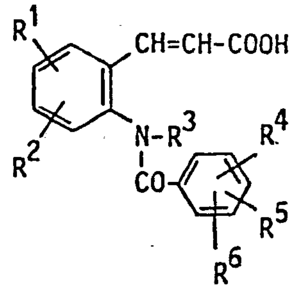

(V),

wherein

$R^1$, $R^2$, $R^3$, $R^4$, $R^5$ and $R^6$ have the meaning stated above, or a salt or a lower alkyl ester thereof, is hydrogenated and, where appropriate, ω-[2-(N-lower alkyl-benzamido)-phenyl]-propionic acid lower alkyl esters obtained are subsequently converted into the free acids or their salts.

8. Process for the preparation of ω-[2-(N-lower alkyl-benzamido)-phenyl]-alkanoic acids of the general formula I*

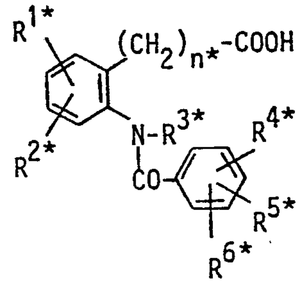

(I*),

wherein

n* denotes a positive whole number from 2 to 5,

$R^{1*}$ denotes a hydrogen atom, a chlorine atom, a bromine atom, a lower alkyl group, a methoxy group or a phenyl group,

$R^{2*}$ denotes a hydrogen atom, a chlorine atom, a bromine atom, a methyl group or a methoxy group,

$R^{3*}$ denotes a straight-chain lower alkyl group,

$R^{4*}$ denotes a hydrogen atom, a fluorine atom, a chlorine atom, a bromine atom, a lower alkyl group, a hydroxyl group, a lower alkoxy group, a phenyl group, a nitro group, an amino group, a dimethylamino group or a trifluoromethyl group,

$R^{5*}$ denotes a hydrogen atom, a chlorine atom, a lower alkyl group or a lower alkoxy group,

$R^{6*}$ denotes a hydrogen atom, a lower alkyl group or a lower alkoxy group,

and their salts with inorganic or organic bases, characterised in that

a) a ω-(2-lower alkylamino-phenyl)-alkanoic acid of the general formula II*

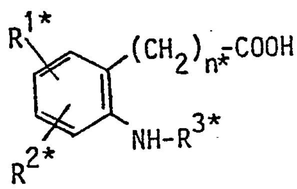

(II*),

wherein

n*, $R^{1*}$, $R^{2*}$ and $R^{3*}$ have the meaning stated above, or a salt thereof, is acylated in basic solution with a benzoyl derivative of the general formula III*

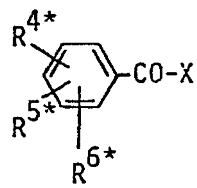

(III*),

wherein

X denotes a halogen atom and $R^{4*}$, $R^{5*}$ and $R^{6*}$ have the meaning stated abvoe, and, if desired, obtained free acids are subsequently converted into their salts, or obtained salts are subsequently converted into the free acids, or

b) a ω-[2-(N-lower alkyl-benzamido)-phenyl]-alkyl-compound of the general formula IV*

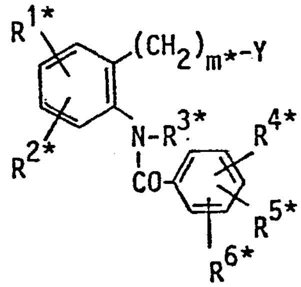

(IV*),

wherein

$R^{1*}$, $R^{2*}$, $R^{3*}$, $R^{4*}$, $R^{5*}$ and $R^{6*}$ have the meaning stated above, m* is a positive whole number from

1 to 4 and Y is a leaving group, is reacted with a dialkyl malonate, preferably with a di-lower-alkylmalonate, in the presence of basic condensation agents, and the resulting substituted malonic ester is saponificated and decarboxylated, and, if desired, obtained free acids are subsequently converted into their salts, or obtained salts are subsequently converted into the free acids, or

c) in order to prepare compounds of the formula I*, wherein n* = 2, a 2-(N-lower alkyl-benzamido)-cinnamic acid of the general formula V*

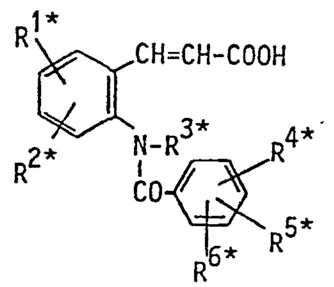

(V*),

wherein

$R^{1*}$, $R^{2*}$, $R^{3*}$, $R^{4*}$, $R^{5*}$ and $R^{6*}$ have the meaning stated above, or a salt or a lower alkyl ester thereof, is hydrogenated and, where appropriate, ω-[2-(N-lower alkylbenzamido)-phenyl]-propionic acid lower alkyl esters obtained are subsequently converted into the free acids or their salts.

9. Process for the preparation of ω-[2-(N-lower alkyl-benzamido)-phenyl]alkanoic acids of the general formula I**

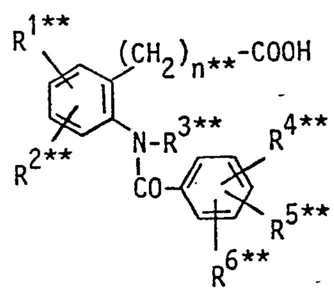

(I**),

wherein

$n^{**}$ denotes a positive whole number from 2 to 5,

$R^{1**}$ denotes a hydrogen atom, a methyl group, a methoxy group, a chlorine atom, a bromine atom or a phenyl group,

$R^{2**}$ denotes a hydrogen atom, a chlorine atom or a methyl group,

$R^{3**}$ denotes a lower alkyl group with 1 to 4 carbon atoms,

$R^{4**}$ denotes a hydrogen atom, a chlorine atom, a bromine atom, a methyl group, a lower alkoxy group, a phenyl group or a trifluoromethyl group,

$R^{5**}$ denotes a hydrogen atom, a chlorine atom, a lower alkyl group or a methoxy group,

$R^{6**}$ denotes a hydrogen atom, a lower alkyl group or a methoxy group, and their salts with inorganic or organic bases, characterised in that

a) a ω-(2-lower alkylamino-phenyl)-alkanoic acid of the general formula II**

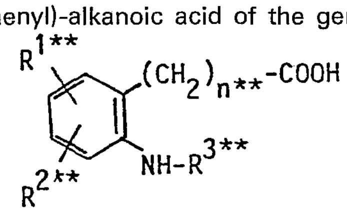

(II**),

wherein

$n^{**}$, $R^{1**}$, $R^{2**}$ and $R^{3**}$ have the meaning stated above, or a salt thereof, is acylated in basic solution with a benzoyl derivative of the general formula III**

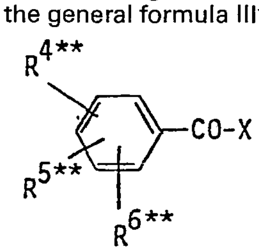

(III**),

wherein

X denotes a halogen atom and

$R^{4**}$, $R^{5**}$ and $R^{6**}$ have the meaning stated above, and, if desired, obtained free acids are subsequently converted into their salts, or obtained salts are subsequently converted into the free acids, or

b) a ω-[2-(N-lower alkyl-benzamido)-phenyl]-alkyl-compound of the general formula IV**

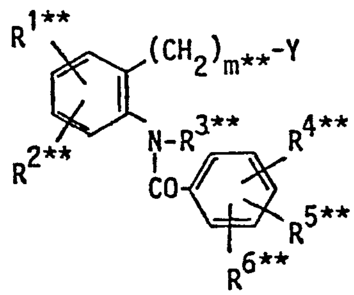

(IV**),

wherein

$R^{1**}$, $R^{2**}$, $R^{3**}$, $R^{4**}$, $R^{5**}$ and $R^{6**}$ have the meaning stated above, m* is a positive whole number from 1 to 4 and Y is a leaving group, is reacted with a dialkyl malonate, preferably with a di-lower alkyl-malonate, in the presence of basic condensation agents, and the resulting substituted malonic ester is saponificated and decarboxylated, and, if desired, obtained free acids are subsequently converted into their salts, or obtained salts are subsequently converted into the free acids,

or

c) in order to prepare compounds of the formula I**, wherein n** = 2, a 2-(N-lower alkyl-benzamido)-cinnamic acid of the general formula V**

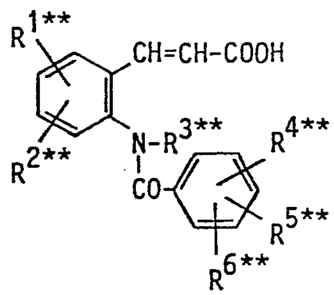

(V**),

wherein

$R^{1**}$, $R^{2**}$, $R^{3**}$, $R^{4**}$, $R^{5**}$ and $R^{6**}$ have the meaning stated above, or a salt or a lower alkyl ester thereof, is hydrogenated and, where appropriate, ω-[2-(N-lower alkyl-benzamido)-phenyl]-propionic acid lower alkyl esters obtained are subsequently converted into the free acids or their salts.

10. Process for the preparation of ω-[2-(N-lower alkyl-benzamido)-phenyl]-alkanoic acids of the general formula I***

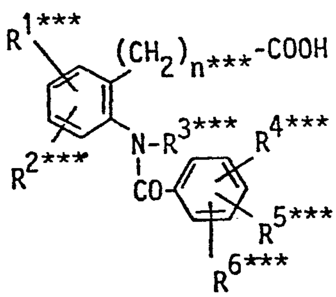

(I***),

wherein

n*** denotes 2 or 3,

$R^{1***}$ denotes a hydrogen atom, a chlorine atom, a methyl group or a methoxy group,

$R^{2***}$ denotes a hydrogen atom or a methyl group,

$R^{3***}$ denotes a methyl group or an ethyl group,

$R^{4***}$ denotes a hydrogen atom, a chlorine atom, a phenyl group or a trifluoromethyl group,

$R^{5***}$ denotes a hydrogen atom or a chlorine atom,

$R^{6***}$ denotes a hydrogen atom, and their pharmacologically compatible salts with inorganic or organic bases, characterised in that

a) a ω-(2-lower alkylamino-phenyl)-alkanoic acid of the general formula II***

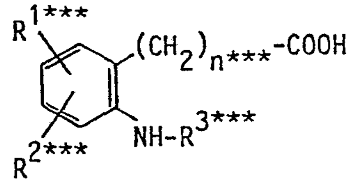

(II*** ),

wherein

n***, R1***, R2*** and R3*** have the meaning stated above, or a salt thereof, is acylated in basic solution with a benzoyl derivative of the general formula III***

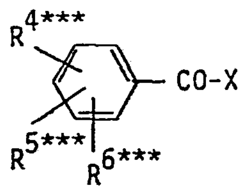

(III***),

wherein

X denotes a halogen atom and R4***, R5*** and R6*** have the meaning stated above, and, if desired, obtained free acids are subsequently converted into their salts, or obtained salts are subsequently converted into the free acids, or

b) a ω-[2-(N-lower alkyl-benzamido)-phenyl]-alkyl-compound of the general formula IV***

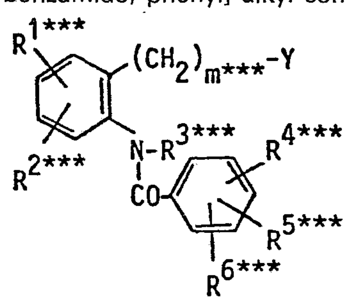

(IV***),

wherein

R1***, R2***, R3***, R4***, R5*** and R6*** have the meaning stated above, m*** is 1 or 2 and Y is a leaving group, is reacted with a dialkyl malonate, preferably with a di-lower-alkylmalonate, in the presence of basic condensation agents, and the resulting substituted malonic ester is saponificated and decarboxylated, and, if desired, obtained free acids are subsequently converted into their salts, or obtained salts are subsequently converted into the free acids, or

c) in order to prepare compounds of the formula I***, wherein n*** = 2, a 2-(N-lower alkyl-benzamido)-cinnamic acid of the general formula V***

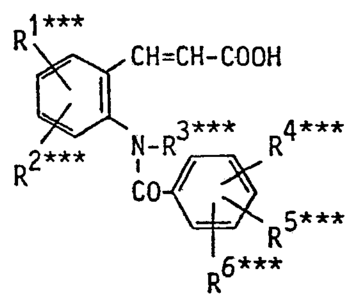

(V***),

wherein

R1***, R2***, R3***, R4***, R5*** and R6*** have the meaning stated above, or a salt or a lower alkyl ester thereof, is hydrogenated and, where appropriate, ω-[2-(N-lower alkyl-benzamido)-phenyl]-propionic acid lower alkyl esters obtained are subsequently converted into the free acids of their salts.

11. Process for the preparation of ω-[2-(N-lower alkyl-benzamido)-phenyl]-alkanoic acids of the general formula I****

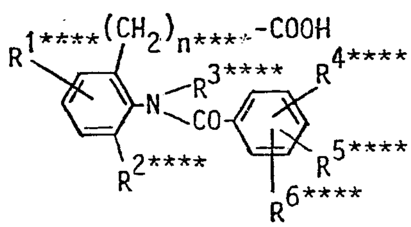

(I****),

wherein

$n^{****}$ denotes 2 or 3,

$R^{1****}$ denotes a hydrogen atom, a chlorine atom, a methyl group or a methoxy group,

$R^{2****}$ denotes a hydrogen atom,

$R^{3****}$ denotes a methyl group,

$R^{4****}$ denotes a hydrogen atom, a chlorine atom or a trifluoromethyl group,

$R^{5****}$ denotes a hydrogen atom or a chlorine atom,

$R^{6****}$ denotes a hydrogen atom, and their pharmacologically compatible salts with inorganic or organic bases, characterised in that

a) a ω-(2-lower alkylamino-phenyl)-alkanoic acid of the general formula $II^{****}$

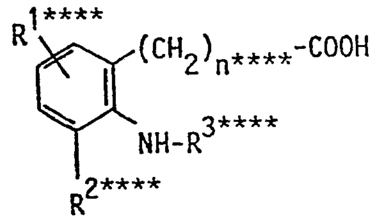

($II^{****}$),

wherein

$n^{****}$, $R^{1****}$, $R^{2****}$ and $R^{3****}$ have the meaning stated above, or a salt thereof, is acylated in basic solution with a benzoyl derivative of the general formula $III^{****}$

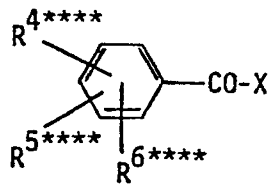

($III^{****}$),

wherein

X denotes a halogen atom and $R^{4****}$, $R^{5****}$ and $R^{6****}$ have the meaning stated above, and, if desired, obtained free acids are subsequently converted into their salts, or obtained salts are subsequently converted into the free acids, or

b) a ω-[2-(N-lower alkyl-benzamido)-phenyl]-alkyl-compound of the general formula $IV^{****}$

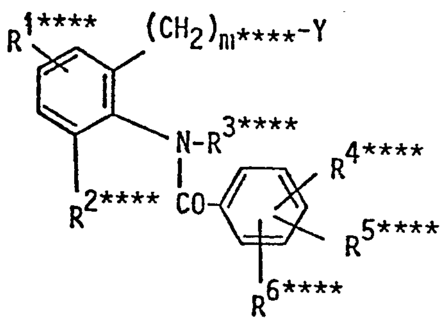

($IV^{****}$),

wherein

$R^{1****}$, $R^{2****}$, $R^{3****}$, $R^{4****}$, $R^{5****}$ and $R^{6****}$ have the meaning stated above, $m^{****}$ is 1 or 2 and Y is a leaving group, is reacted with a dialkyl malonate, preferably with a di-lower-alkylmalonate, in the presence of basic condensation agents, and the resulting substituted malonic ester is saponificated and decarboxylated, and, if desired, obtained free acids are subsequently converted into their salts, or obtained salts are subsequently converted into the free acids, or

c) in order to prepare compounds of the formula $I^{****}$, wherein $n^{****} = 2$, a 2-(N-lower alkyl-benzamido)-cinnamic acid of the general formula $V^{****}$

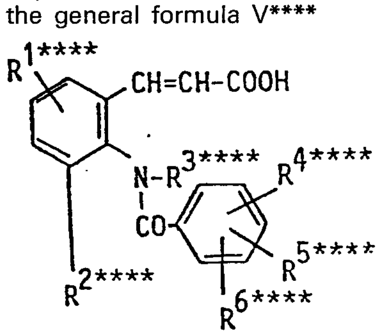

($V^{****}$),

wherein

$R^{1****}$, $R^{2****}$, $R^{3****}$, $R^{4****}$, $R^{5****}$ and $R^{6****}$ have the meaning stated above, or a salt or a lower alkyl ester thereof, is hydrogenated and, where appropriate, ω-[2-(N-lower alkyl-benzamido)-phenyl]-propionic acid lower alkyl esters obtained are subsequently converted into the free acids or their salts.

12. Compounds according to claims 1 to 5 for the treatment and prophylaxis of diseases which are based on disturbances of glucose metabolism.

13. Use of the compounds according to claims 1 to 5 in the preparation of medicaments which are used for the treatment and prophylaxis of diseases which are based on disturbances of glucose metabolism.

**Revendications**

1. Acides ω-[2-(N-alkyl inférieur-benzamido)-phényl]-alcanoïques de formule générale I:

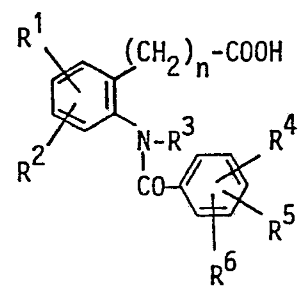

(I),

dans laquelle

n est un nombre positif entier de 2 à 5,

$R^1$ représente un atome d'hydrogène, un atome d'halogène, un groupe alkyle inférieur, alcoxy inférieur, alkyl inférieur-mercapto, trifluorométhyle ou phényle éventuellement substitué par un atome d'halogène ou par un groupe alcoxy inférieur,

$R^2$ représente un atome d'hydrogène, un atome d'halogène, un groupe alkyle inférieur ou alcoxy inférieur,

$R^3$ représente un groupe alkyle inférieur,

$R^4$ représente un atome d'hydrogène, un atome d'halogène, un groupe hydroxy, alkyle inférieur, alcoxy inférieur, alkyl inférieur-mercapto, phényle éventuellement substitué par un atome d'halogène ou par un groupe nitro, un groupe nitro, un groupe amino éventuellement substitué par un groupe alkyle inférieur, un groupe alkyl inférieur-carbonyle, un groupe benzoyle éventuellement substitué par un atome d'halogène, un groupe trifluorométhyle, trifluorométhoxy ou trifluorométhylmercapto,

$R^5$ représente un atome d'hydrogène, un atome d'halogène, un groupe alkyle inférieur ou alcoxy inférieur,

$R^6$ représente un atome d'hydrogène, un groupe alkyle inférieur ou alcoxy inférieur,

de même que leurs sels avec des bases organiques ou inorganiques.

2. Acides ω-[2-(N-alkyl inférieur-benzamido)-phényl]-alcanoïques de formule générale I*:

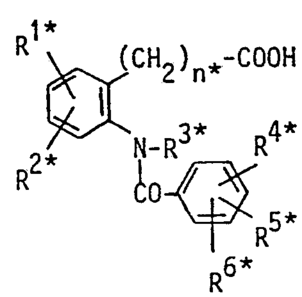

(I*),

dans laquelle

$n^*$ représente un nombre positif entier de 2 à 5,

$R^{1*}$ représente un atome d'hydrogène, un atome de chlore, un atome de brome, un groupe alkyle inférieur, methoxy ou phényle,

$R^{2*}$ représente un atome d'hydrogène, un atome de chlore, un atome de brome, un groupe méthyle ou méthoxy,

$R^{3*}$ représente un groupe alkyle inférieur à chaîne droite,

$R^{4*}$ représente un atome d'hydrogène, un atome de fluor, un atome de chlore, un atome de brome, un groupe alkyle inférieur, hydroxy, alcoxy inférieur, phényle, nitro, amino, diméthylamino ou trifluorométhyle,

R[5*] représente un atome d'hydrogène, un atome de chlore, un groupe alkyle inférieur ou alcoxy inférieur,

R[6*] représente un atome d'hydrogène, un groupe alkyle inférieur ou alcoxy inférieur,

de même que leurs sels avec des bases organiques ou inorganiques.

3. Acides ω-[2-(N-alkyl inférieur-benzamido)-phényl]-alcanoïques de formule générale I**:

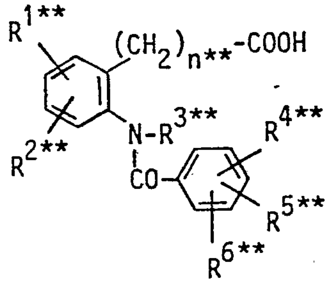

(I**),

dans laquelle

n** représente un nombre positif entier de 2 à 5,

R[1**] représente un atome d'hydrogène, un groupe méthyle, un groupe méthoxy, un atome de chlore, un atome de brome ou un groupe phényle,

R[2**] représente un atome d'hydrogène, un atome de chlore ou un groupe méthyle,

R[3**] représente un groupe alkyle à chaîne droite contenant 1 à 4 atomes de carbone,

R[4**] représente un atome d'hydrogène, un atome de chlore, un atome de brome, un groupe méthyle, un groupe alcoxy inférieur, un groupe phényle ou un groupe trifluorométhyle,

R[5**] représente un atome d'hydrogène, un atome de chlore, un groupe alkyle inférieur ou un groupe méthoxy,

R[6**] représente un atome d'hydrogène, un groupe alkyle inférieur ou un groupe méthoxy,

de même que leurs sels avec des bases organiques ou inorganiques.

4. Acides ω-[2-(N-alkyl inférieur-benzamido)-phényl]-alcanoïques de formule générale I***:

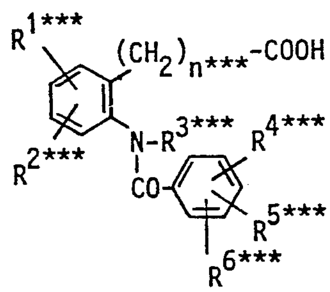

(I***),

dans laquelle

n*** représente 2 ou 3,

R[1***] représente un atome d'hydrogène, un atome de chlore, un groupe méthyle ou un groupe méthoxy,

R[2***] représente un atome d'hydrogène ou un groupe méthyle,

R[3***] représente un groupe méthyle ou éthyle,

R[4***] représente un atome d'hydrogène, un atome de chlore, un groupe phényle ou un groupe trifluorométhyle,

R[5***] représente un atome d'hydrogène ou un atome de chlore,

R[6***] représente un atome d'hydrogène,

de même que leurs sels pharmacologiquement compatibles avec des bases organiques ou inorganiques.

5. Acides ω-[2-(N-alkyl inférieur-benzamido)-phényl-alcanoïques de formule générale I****:

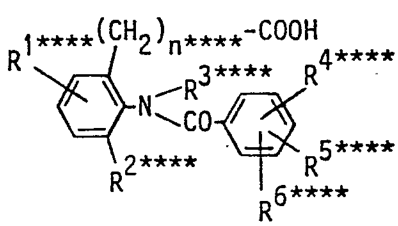

(I****),

dans laquelle

n**** représente 2 ou 3,

R[1****] représente un atome d'hydrogène, un atome de chlore, un groupe méthyle ou un groupe méthoxy,

$R^{2****}$ représente un atome d'hydrogène,
$R^{3****}$ représente un group méthyle,
$R^{4****}$ représente un atome d'hydrogène, un atome de chlore ou un groupe trifluorométhyle,
$R^{5****}$ représente un atome d'hydrogène ou un atome de chlore,
$R^{6****}$ représente un atome d'hydrogène,
de même que leurs sels pharmacologiquement compatibles avec des bases organiques ou inorganiques.

6. Médicament contenant un ou plusieurs composés suivant les revendications 1 à 5.

7. Procédé de préparation d'acides $\omega$-[2-(N-alkyl inférieur-benzamido)-phényl]-alcanoïques de formule générale I:

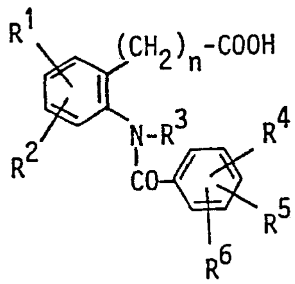

(I),

dans laquelle
n est un nombre positif entier de 2 à 5,
$R^1$ représente un atome d'hydrogène, un atome d'halogène, un groupe alkyle inférieur, alcoxy inférieur, alkyl inférieur-mercapto, trifluorométhyle ou un groupe phényle éventuellement substitué par un atome d'halogène ou par un groupe alcoxy inférieur,
$R^2$ représente un atome d'hydrogène, un atome d'halogène, un groupe alkyle inférieur ou un groupe alcoxy inférieur,
$R^3$ représente un groupe alkyle inférieur,
$R^4$ représente un atome d'hydrogène, un atome d'halogène, un groupe hydroxy, un groupe alkyle inférieur, un groupe alcoxy inférieur, un groupe alkyl inférieur-mercapto, un groupe phényle éventuellement substitué par un atome d'halogène ou par un groupe nitro, un groupe nitro, un groupe amino éventuellement substitué par un groupe alkyle inférieur, un groupe alkyl inférieur-carbonyle, un groupe benzoyle éventuellement substitué par un atome d'halogène, un groupe trifluorométhyle, un groupe trifluorométhoxy ou un groupe trifluorométhylmercapto,
$R^5$ représente un atome d'hydrogène, un atome d'halogène, un groupe alkyle inférieur ou un groupe alcoxy inférieur,
$R^6$ représente un atome d'hydrogène, un groupe alkyle inférieur ou un groupe alcoxy inférieur,
ainsi que de leurs sels avec des bases organiques ou inorganiques, caractérisé en ce que:

a) on soumet, à une acylation en solution basique, un acide $\omega$-(2-alkyl inférieur-amino-phényl)-alcanoïque de formule générale II:

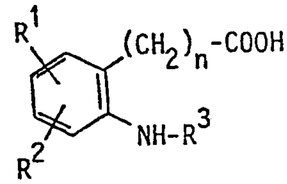

(II),

dans laquelle
n, $R^1$, $R^2$ et $R^3$ ont les significations indiquées ci-dessus, ou un de ses sels, avec un dérivé benzoyle de formule générale III:

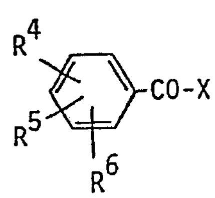

(III),

dans laquelle
X représente un atome d'halogène, tandis que $R^4$, $R^5$ et $R^6$ ont les significations indiquées ci-dessus et, si on le désire, on transforme ensuite les acides libres obtenus en leurs sels ou les sels obtenus en acides libres, ou

b) on fait réagir un composé ω-[2-(N-alkyl inférieur-benzamido)-phényl]-alkylique de formule générale IV:

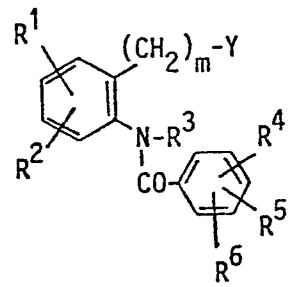

(IV),

dans laquelle

$R^1$, $R^2$, $R^3$, $R^4$, $R^5$ et $R^6$ ont les significations indiquées ci-dessus, m représente un nombre positif entier de 1 à 4 et Y représente un groupe qui s'éloigne, avec un malonate de dialkyle, de préférence, un malonate de dialkyle inférieur, en présence d'agents de condensation basiques, puis on soumet l'ester malonique substitué obtenu à une saponification et à une décarboxylation et, si on le désire, on transforme ensuite les acides libres obtenus en leurs sels ou les sels obtenus en acides libres, ou

c) pour préparer des composés de formule I dans laquelle n = 2, on hydrogène un acide 2-(N-alkyl inférieur-benzamido)-cinnamique de formule générale V:

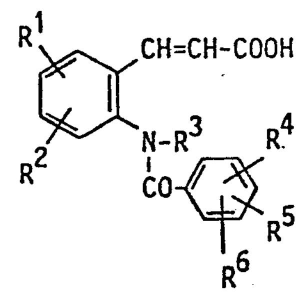

(V),

dans laquelle

$R^1$, $R^2$, $R^3$, $R^4$, $R^5$ et $R^6$ ont les significations indiquées ci-dessus, ou un de ses sels ou de ses esters alkyliques inférieurs et l'on transforme ensuite éventuellement les esters alkyliques inférieures d'acides ω-[2-(N-alkyl inférieur-benzamido)-phényl]-propioniques obtenus en acides libres ou en leurs sels.

8. Procédé de préparation d'acides ω-[2-(N-alkyl inférieur-benzamido)-phényl]-alcanoïques de formule générale I*:

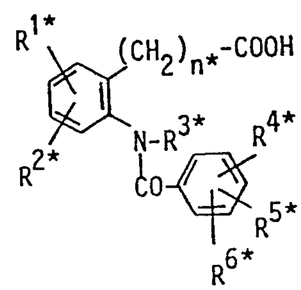

(I*),

dans laquelle

- n* représente un nombre positif entier de 2 à 5,
- $R^{1*}$ représente un atome d'hydrogène, un atome de chlore, un atome de brome, un groupe alkyle inférieur, un groupe méthoxy ou un groupe phényle,
- $R^{2*}$ représente un atome d'hydrogène, un atome de chlore, un atome de brome, un groupe méthyle ou un groupe méthoxy,
- $R^{3*}$ représente un groupe alkyle inférieur à chaîne droite,
- $R^{4*}$ représente un atome d'hydrogène, un atome de fluor, un atome de chlore, un atome de brome, un groupe alkyle inférieur, hydroxy, alcoxy inférieur, phényle, nitro, amino, diméthylamino ou trifluorométhyle,
- $R^{5*}$ représente un atome d'hydrogène, un atome de chlore, un groupe alkyle inférieur ou alcoxy inférieur,
- $R^{6*}$ représente un atome d'hydrogène, un groupe alkyle inférieur ou alcoxy inférieur,

ainsi que de leurs sels avec des bases organiques ou inorganiques, caractérisé en ce que:

a) on soumet, à une acylation en solution basique, un acids $\omega$-(2-alkyl inférieur-amino-phényl)-alcanoïque de formule générale II*:

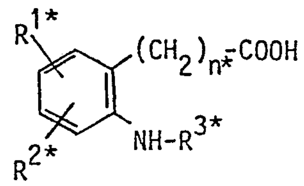

(II*),

dans laquelle

n*, R¹*, R²* et R³* ont les significations indiquées ci-dessus, ou un de ses sels, avec un dérivé benzoyle de formule générale III*:

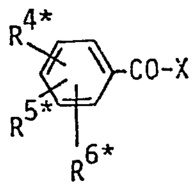

(III*),

dans laquelle

X représente un atome d'halogène, tandis que R⁴*, R⁵* et R⁶* ont les significations indiquées ci-dessus et, si on le désire, on transforme éventuellement les acides libres obtenus en leurs sels ou les sels obtenus en acides libres, ou

b) on fait réagir un composé $\omega$-[2-(N-alkyl inférieur-benzamido)-phényl]-alkylique de formule générale IV*:

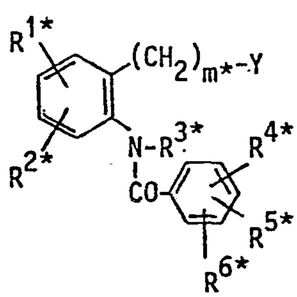

(IV*),

dans laquelle

R¹*, R²*, R³*, R⁴*, R⁵* et R⁶* ont les significations indiquées ci-dessus, m* représente un nombre positif entier de 1 à 4 et Y représente un groupe qui s'éloigne, avec un malonate de dialkyle, de préférence, un malonate de dialkyle inférieur, en présence d'agents de condensation basiques et on soumet l'ester malonique substitué obtenu à une saponification et à une décarboxylation et, si on le désire, on transforme ensuite les acides libres obtenus en leurs sels ou les sels obtenus en acides libres, ou

c) pour préparer des composés de formule I* dans laquelle n* = 2, on hydrogène un acide 2-(N-alkyl inférieur-benzamido)-cinnamique de formule générale V*:

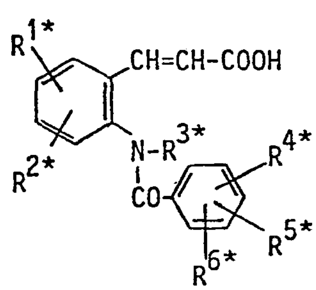

(V*),

dans laquelle

R¹*, R²*, R³*, R⁴*, R⁵* et R⁶* ont les significations indiquées ci-dessus, ou un de ses sels ou de ses esters alkyliques inférieurs et l'on transforme éventuellement ensuite les esters alkyliques inférieurs d'acides $\omega$-[2-(N-alkyl inférieur-benzamido)-phényl]-propioniques obtenus en acides libres ou en leurs sels.

9. Procédé de préparation d'acides $\omega$-[2-(N-alkyl inférieur-benzamido)-phényl]-alcanoïques de formule générale I**:

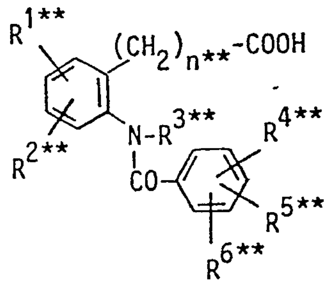

(I**),

dans laquelle

n** représente un nombre positif entier de 2 à 5,

R1** représente un atome d'hydrogène, un groupe méthyle, un groupe méthoxy, un atome de chlore, un atome de brome ou un groupe phényle,

R2** représente un atome d'hydrogène, un atome de chlore ou un groupe méthyle,

R3** représente un groupe alkyle à chaîne droite contenant 1 à 4 atomes de carbone,

R4** représente un atome d'hydrogène, un atome de chlore, un atome de brome, un groupe méthyle, un groupe alcoxy inférieur, un groupe phényle ou un groupe trifluorométhyle,

R5** représente un atome d'hydrogène, un atome de chlore, un groupe alkyle inférieur ou un groupe méthoxy,

R6** représente un atome d'hydrogène, un groupe alkyle inférieur ou un groupe méthoxy, ainsi que de leurs sels avec des bases organiques ou inorganiques, caractérisé en ce que:

a) on soumet, à une acylation en solution basique, un acide $\omega$-(2-alkyl inférieur-amino-phényl)-alcanoïque de formule II**:

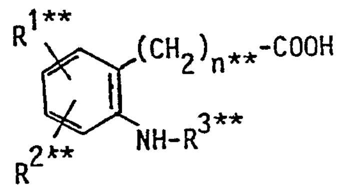

(II**),

dans laquelle

n**, R1**, R2** et R3** ont les significations indiquées ci-dessus, ou un de ses sels avec un dérivé benzoyle de formule générale III**:

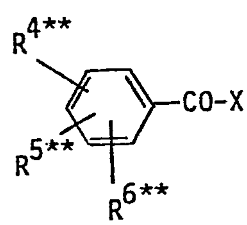

(III**),

dans laquelle

X représente un atome d'halogène, tandis que R4**, R5** et R6** ont les significations indiquées ci-dessus et, si on le désire, on transforme ensuite les acides libres obtenus en leurs sels ou les sels obtenus en acides libres, ou

b) on fait réagir un composé $\omega$-[2-(N-alkyl inférieur-benzamido)-phényl]-alkylique de formule générale IV**:

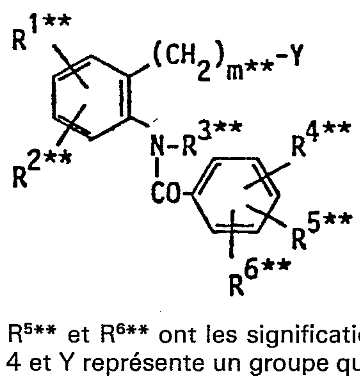

(IV**),

dans laquelle

R1**, R2**, R3**, R4**, R5** et R6** ont les significations indiquées ci-dessus, m** représente un nombre positif entier de 1 à 4 et Y représente un groupe qui s'éloigne, avec un malonate de dialkyle, de

préférence, un malonate de dialkyle inférieur, en présence d'agents de condensation basiques, puis on soumet l'ester malonique substitué obtenu à une saponification et à une décarboxylation et, si on le désire, on transforme ensuite les acides libres obtenus en leurs sels ou les sels obtenus en acides libres, ou

c) pour préparer des composés de formule I** dans laquelle n** = 2, on hydrogène un acide 2-(N-alkyl inférieur-benzamido)-cinnamique de formule générale V**:

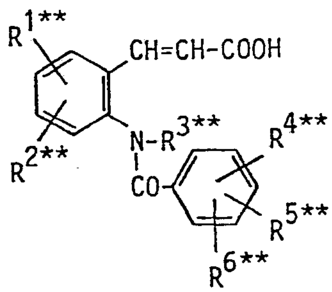

(V**),

dans laquelle

R¹**, R²**, R³**, R⁴**, R⁵** et R⁶** ont les significations indiquées ci-dessus, ou un de ses sels ou de ses esters alkyliques inférieurs, et on transforme éventuellement ensuite les esters alkyliques inférieurs d'acides $\omega$-[2-(N-alkyl inférieur-benzamido)-phényl]-propioniques obtenus en acides libres ou leurs sels.

10. Procédé de préparation d'acides $\omega$-[2-(N-alkyl inférieur-benzamido)-phényl]-alcanoïques de formule générale I***:

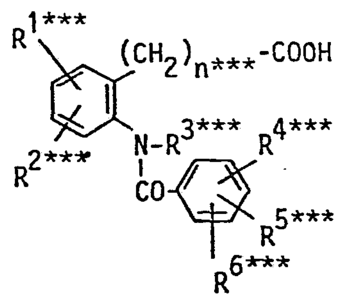

(I***),

dans laquelle

n*** représente 2 ou 3,
$R^{1***}$ représente un atome d'hydrogène, un atome de chlore, un groupe méthyle ou un gorupe méthoxy,
$R^{2***}$ représente un atome d'hydrogène ou un groupe méthyle,
$R^{3***}$ représente un groupe méthyle ou un groupe éthyle,
$R^{4***}$ représente un atome d'hydrogène, un atome de chlore, un groupe phényle ou un groupe trifluorométhyle,
$R^{5***}$ représente un atome d'hydrogène ou un atome de chlore,
$R^{6***}$ représente un atome d'hydrogène,

ainsi que de leurs sels pharmacologiquement compatibles avec des bases organiques ou inorganiques, caractérisé en ce que:

a) on soumet, à une acylation en solution basique, un acide $\omega$-(2-alkyl inférieur-amino-phényl)-alcanoïque de formule générale II***:

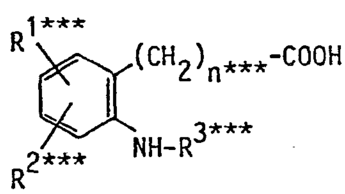

(II***),

dans laquelle

n***, $R^{1***}$, $R^{2***}$ et $R^{3***}$ ont les significations indiquées ci-dessus, ou un de ses sels, avec un dérivé benzoyle de formule générale III***:

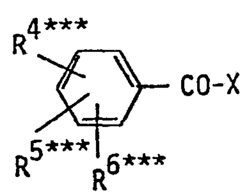

(III***),

dans laquelle

X représente un atome d'halogène, tandis que $R^{4***}$, $R^{5***}$ et $R^{6***}$ ont les significations indiquées ci-dessus et, si on le désire, on transforme ensuite les acides libres obtenus en leurs sels ou les sels obtenus en acides libres, ou

b) on fait réagir un composé ω-[2-(N-alkyl inférieur-benzamido)-phényl]-alkylique de formule générale IV***:

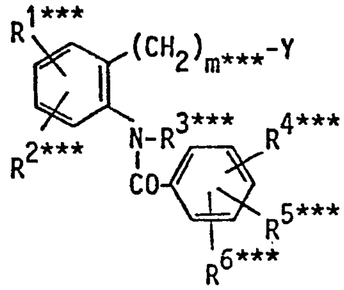

(IV***),

dans laquelle

$R^{1***}$, $R^{2***}$, $R^{3***}$, $R^{4***}$, $R^{5***}$ et $R^{6***}$ ont les significations indiquées ci-dessus, m*** représente 1 ou 2 et Y représente un groupe qui s'éloigne, avec un malonate de dialkyle, de préférence, un malonate de dialkyle inférieur, en présence d'agents de condensation basiques, puis on soumet l'ester malonique substitué obtenu à une saponification et à une décarboxylation et, si on le désire, on transforme ensuite les acides libres obtenus en leurs sels ou les sels obtenus en acides libres, ou

c) pour préparer des composés de formule I*** dans laquelle n*** = 2, on hydrogène une acide 2-(N-alkyl inférieur-benzamido)-cinnamique de formule générale V***:

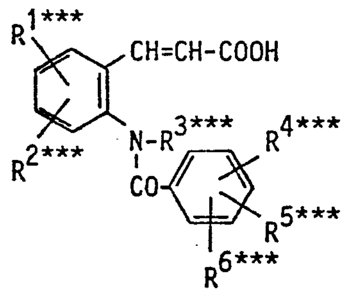

(V***),

dans laquelle

$R^{1***}$, $R^{2***}$, $R^{3***}$, $R^{4***}$, $R^{5***}$ et $R^{6***}$ ont les significations indiquées ci-dessus, ou un de ses sels ou de ses esters alkyliques inférieurs et on transforme éventuellement ensuite les esters alkyliques inférieurs d'acides ω-[2-(N-alkyl inférieur-benzamido)phényl]-propioniques obtenus en acides libres ou leurs sels.

11. Procédé de préparation d'acides ω-[2-(N-alkyl inférieur-benzamido)-phényl]-alcanoïques de formule générale I****:

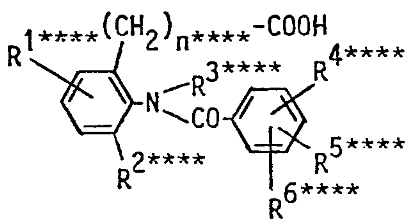

(I****),

dans laquelle

n**** représente 2 ou 3,

$R^{1****}$ représente un atome d'hydrogène, un atome de chlore, un groupe méthyle ou un groupe méthoxy,

$R^{2****}$ représente un atome d'hydrogène,

$R^{3****}$ représente un groupe méthyle,

$R^{4****}$ représente un atome d'hydrogène, un atome de chlore ou un groupe trifluorométhyle,

$R^{5****}$ représente un atome d'hydrogène ou un atome de chlore,

$R^{6****}$ représente un atome d'hydrogène,

ainsi que de leurs sels pharmacologiquement compatibles avec des bases organiques ou inorganiques, caractérisé en ce que:

a) on soumet, à une acylation en solution baque, un acide ω-(2-alkyl inférieur-amino-phényl)-alcanoïque de formule générale II****:

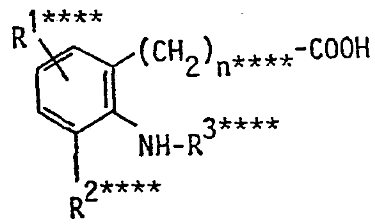

(II****),

dans laquelle

$n^{****}$, $R^{1****}$, $R^{2****}$ et $R^{3****}$ ont les significations indiquées ci-dessus, ou un de ses sels, avec un dérivé benzoyle de formule générale III****:

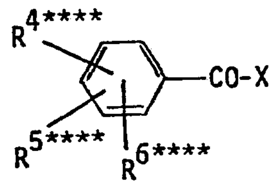

(III****),

dans laquelle

X représente un atome d'halogène, tandis que $R^{4****}$, $R^{5****}$ et $R^{6****}$ ont les significations indiquées ci-dessus et, si on le désire, on transforme ensuite les acides libres obtenus en leurs sels ou les sels obtenus en acides libres, ou

b) on fait réagir un composé ω-[2-(N-alkyl inférieur-benzamido)-phényl]-alkylique de formule générale IV****:

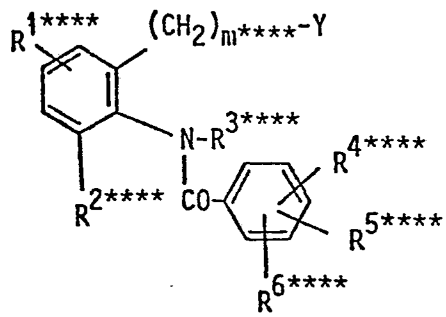

(IV****),

dans laquelle

$R^{1****}$, $R^{2****}$, $R^{3****}$, $R^{4****}$, $R^{5****}$ et $R^{6****}$ ont les significations indiquées ci-dessus, $m^{****}$ représente 1 ou 2 et Y représente un groupe qui s'éloigne, avec un malonate de dialkyle, de préférence, un malonate de dialkyle inférieur, en présence d'agents de condensation basiques, puis on soumet l'ester malonique substitué obtenu à une saponification et à une décarboxylation et, si on le désire, on transforme ensuite les acides libres obtenus en leurs sels ou les sels obtenus en acides libres, ou

c) pour préparer des composés de formule I**** dans laquelle $n^{****} = 2$, on hydrogène un acide 2-(N-alkyl inférieur-benzamido)-cinnamique de formule générale V****:

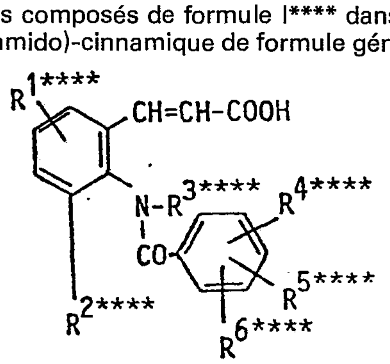

(V****),

dans laquelle $R^{1****}$, $R^{2****}$, $R^{3****}$, $R^{4****}$, $R^{5****}$ et $R^{6****}$ ont les significations indiquées ci-dessus, ou un de ses sels ou de ses esters alkyliques inférieurs et on transforme éventuellement ensuite les esters alkyliques inférieurs d'acides ω-[2-(N-alkyl inférieur-benzamido)-phényl]-propioniques obtenus en acides libres ou leurs sels.

12. Composés suivant les revendications 1 à 5 utilisés pour le traitement et la prophylaxie de maladies reposant sur les perturbations du métabolisme du glucose.

13. Utilisation de composés suivant les revendications 1 à 5 pour la préparation de médicaments utilisés pour le traitement et la prophylaxie de maladies reposant sur les perturbations du métabolisme du glucose.